# EUROPEAN PATENT APPLICATION

(11) **EP 2 361 549 A2**
(43) Date of publication of application: **31.08.2011**
(21) Application number: 11157552.8
(22) Date of filing: 29.09.2006
(51) Int. Cl.: A61B 5/00, A61F 2/06, A61M 25/10

(54) **System for analysis and treatment of a body lumen**

(30) Priority: 30.09.2005 US 722753 P; 24.01.2006 US 761649 P; 07.08.2006 US 821623 P; 29.08.2006 US 823812 P; 08.09.2006 US 824915 P
(62) Divisional of application: 06816081.1
(71) Applicant: Cornova, Inc., Burlington, MA 01803 (US)
(72) Inventor: Ryan, Eric S., Hopkinton, MA 01748 (US); Tang, Jing, Arlington, MA 02472 (US)
(74) Representative: Lane, Cathal Michael

(57) **Abstract**

In a system and method for analyzing and treating a body lumen, a lumen-expanding balloon catheter with integrated one or more delivery waveguides and one or more collection waveguides is used to perform optical analysis of the tissues surrounding the lumen during expansion. The catheter can comprise an angioplasty catheter with integrated delivery waveguides and collection waveguides to perform spectroscopy of a stenotic plaque during angioplasty.

## Description

### RELATED APPLICATIONS

This application claims the benefit of U.S. Provisional Patent Application Number 60/722,753 filed on September 30, 2005, U.S. Provisional Patent Application Number 60/761,649 filed on January 24, 2006,U.S. Provisional Patent Application Number 60/821,623 filed on August 7, 2006, U.S. Provisional Patent Application Number 60/823,812 filed on August 29, 2006, and U.S. Provisional Patent Application Number 60/824,915 filed on September 8, 2006, the contents of each being incorporated herein by reference.

### BACKGROUND OF THE INVENTION

### 1. Field of the Invention

Embodiments of the present invention are directed to systems and methods for the analysis and treatment of a lumen. More particularly, the present invention relates to a balloon catheter that is used to perform an angioplasty of endovascular lesions and a method of treatment using such a balloon catheter.

### 2. Description of the Related Art

With the continual expansion of minimally-invasive procedures in medicine, one procedure that has been highlighted in recent years has been percutaneous transluminal angioplasty, or "PTA". The most prevalent use of this procedure is in the coronary arteries, which is more specifically called a percutaneous coronary transluminal angioplasty, or "PTCA". These procedures utilize a flexible catheter with an inflation lumen to expand, under relatively high pressure, a balloon at the distal end of the catheter to expand a stenotic lesion.

The PTA and PTCA procedures are now commonly used in conjunction with expandable tubular structures known as stents and an angioplasty balloon is often used to expand and permanently place the stent within the lumen. An angioplasty balloon utilized with a stent is referred to as a stent delivery system. Conventional stents have been shown to be more effective than angioplasty alone in maintaining patency in most types of lesions and also reducing other near-term endovascular events. A risk with a conventional stent, however, is the reduction in efficacy of the stent due to the growth of the tissues surrounding the stent which can again result in the stenosis of the lumen, often referred to as restenosis. In recent years, new stents that are coated with a pharmaceutical agents, often in combination with a polymer, have been introduced and shown to significantly reduce the rate of restenosis. These coated stents are generally referred to as drug-eluting stents, though some coated stents have a passive coating instead of an active pharmaceutical agent.

With the advent of these advanced technologies for PTA and PTCA, there has been a substantial amount of clinical and pathology literature published about the pathophysiologic or morphologic factors within an endovascular lesion that contribute to its restenosis or other acute events such as thrombosis. These features include, but are not limited to, collagen content, lipid content, calcium content, inflammatory factors, and the relative positioning of these features within the plaque. Several studies have been provided showing the promise of identifying the above factors through the use of visible and/or near infrared spectroscopy (i.e. across wavelengths ranging between about 250 to 2500 nm), including those studies referenced in U.S. Publication No. US2004/0111016A1 by Casscells, III et al., U.S. Publication No. US2004/0077950A1 by Marshik-Geurts et al., U.S. Patent No. 5,304,173 by Kittrell et al., and U.S. Patent No. 6,095,982 by Richards-Kortum, et al., the contents of each of which are herein incorporated by reference. However, there are very few, if any, highly safe and commercially viable applications making use of this spectroscopic data for combining diagnosis and treatment in a PTA or PTCA procedure.

Unfortunately, the most common diagnostic procedure associated with PTA or PTCA is angiography by fluoroscopy. This X-ray technology simply supplies an image of the blood flow within a lumen, thus identifying a stenosis, but giving no information about the endovascular wall of the plaque. Some important diseases located on non- or minor stenosis regions, such as a vulnerable plaque which is fatal to a patient life, are often missed. Other technologies, such as intravascular ultrasound, require expensive additional catheters and potentially dangerous additional procedures that can cause more harm than good and still not supply sufficient information about the plaque to be beneficial. There is currently no option for physicians to gain this useful information about the lumen wall in an accurate, cost-effective, and efficient manner that presents a reasonable risk profile for the patient.

Conventional balloon catheters suffer from a number of shortcomings and are used for other purposes than analysis of the pathophysiologic or morphologic features of the lumen wall at the lumen-expansion site. Prior use of optical fibers within an angioplasty catheter permit functions such as visualization to occur, but no optical analysis is obtained. Conventional balloon catheters therefore have no capacity to collect any information beyond the surface of the endovascular wall. While lower-pressure balloon catheters are available to occlude the blood flow proximal to the optical analysis window of a catheter, no lumen expansion is performed and no analysis can be performed within the balloon itself. Other systems support the use of optical feedback within a balloon catheter to atraumatically minimize the blood path between the balloon catheter and the endovascular wall. However, these systems likewise provide no ability to perform a complete optical analysis of the lumen wall.

### SUMMARY OF THE INVENTION

The systems and methods described in the present specification provide physicians performing a lumen-expansion procedure with very useful information about the lumen wall without any significant increase in their procedure time or cost, and with little to no additional risk to the patient. Included are a number of implementations of the distal fiber-optic configuration to optimally facilitate illumination of the lumen wall and collection of resultant optical signal. These implementations also provide manufacturability and relatively low-cost production required for a disposable medical device.

In accordance with aspects of the invention, there are provided systems and methods that perform both a lumen expansion and optical analysis of a lumen wall. In one embodiment, the apparatus comprises a lumen-expanding balloon catheter having one or more delivery waveguides and one or more collection waveguides to perform optical analysis of the tissues surrounding the lumen undergoing expansion. In this manner, the delivery and collection of optical radiation is performed within the balloon of the catheter itself. Preferably, the optical analysis is performed when the lumen-expanding balloon is fully inflated, facilitating a relatively unobstructed optical path to the expanded lumen wall. Upon performing the lumen-expanding procedure and optical analysis, a computer can be utilized to analyze the optical signal to provide pathophysiologic or morphologic information about the lumen wall and thus guide appropriate additional treatment.

In one embodiment, optical analysis of the plaque is performed within the same catheter utilized for angioplasty during a PTA or PTCA procedure. This optical analysis could include, but not limited to, Raman spectroscopy, infrared spectroscopy, fluorescence spectroscopy, optical coherence reflectometery, optical coherence tomography, but most preferably diffuse-reflective, near-infrared spectroscopy. The embodiment provides optical analysis, and thus the pathophysiologic or morphologic features diagnosis, of a plaque during an angioplasty procedure without any significant additional cost, risk, or work for the physician. With access to this information, a physician could potentially choose from a selection of drug-eluting stents with different doses or agents, or even select a stent without a drug if indicated. By performing multiple angioplasties during a single visit by a patient, a physician could learn more about the general status of the patient's vasculature which can guide systemic therapies. New emerging technologies such as bioabsorbable stents could be enabled by the embodiments of the invention to optimize their use in the correct type of lesion.

Other advantages and novel features, including optical methods and designs of illuminating and collecting an optical signal of a lumen wall through a lumen-expanding balloon, are described within the detailed description of the various embodiments of the present specification.

In one aspect, a catheter for placement within a body lumen comprises: a flexible conduit that is elongated along a longitudinal axis, the flexible conduit having a proximal end and a distal end; at least one delivery waveguide and at least one collection waveguide positioned along the flexible conduit, the at least one delivery waveguide and the at least one collection waveguide constructed and arranged to transmit radiation at a wavelength in a range of about 250 to 2500 nanometers; and a lumen-expanding inflatable balloon disposed about a portion of the conduit, a transmission output of the at least one delivery waveguide and a transmission input of the at least one collection waveguide located within the balloon.

In one embodiment, the lumen-expanding balloon can comprise an angioplasty balloon.

In another embodiment, the transmission output of the at least one delivery waveguide and the transmission input of the at least one collection waveguide are contiguously retained to the flexible conduit.

In another embodiment, the catheter further comprises a fiber holder disposed about the conduit that contiguously retains the transmission output of the at least one delivery waveguide and the transmission input of the at least one collection waveguide to the flexible conduit.

In another embodiment, the fiber holder comprises at least one holder body having a plurality of holes that are substantially aligned with the longitudinal axis of the conduit when mounted thereto, the at least one delivery waveguide and the at least one collection waveguide being secured to the fiber holder at the holes.

In another embodiment, the fiber holder comprises at least one holder body having a plurality of grooves on a surface thereof, the at least one delivery waveguide and the at least one collection waveguide being secured to the fiber holder at the grooves.

In another embodiment, the plurality of grooves are arranged in a helix.

In another embodiment, the plurality of grooves are substantially aligned with the longitudinal axis of the conduit when the fiber holder is mounted thereto.

In another embodiment, the fiber holder is longitudinally translatable relative to the longitudinal axis of the flexible conduit so that the transmission output of the at least one delivery waveguide and the transmission input of the at least one collection waveguide are translatable between a first longitudinal position and a second longitudinal position of the conduit.

In another embodiment, the fiber holder is rotatable about the longitudinal axis of the flexible conduit so that the transmission output of the at least one delivery waveguide and the transmission input of the at least one collection waveguide can be rotated about the conduit.

In another embodiment, the transmission output of the at least one delivery waveguide and the transmission input of the at least one collection waveguide are spaced apart at a predetermined distance in order to facilitate collection of radiation emitted from tissue of a predetermined depth from the lumen-expanding inflatable balloon through the transmission input.

In another embodiment, the at least one delivery waveguide comprises at least one delivery fiber optic and wherein the at least one collection waveguide comprises at least one collection fiber optic.

In another embodiment, the at least one delivery fiber optic has a tapered end that operates as a reflection surface for changing a direction of a path of radiation transmitted along a longitudinal axis of the delivery fiber optic so that the radiation is emitted in a direction that is transverse to the longitudinal axis of the fiber.

In another embodiment, the at least one collection fiber optic has a tapered end that operates as a reflection surface for changing a direction of a path of radiation transmitted into the transmission input of the collection fiber optic so that the radiation is transmitted along a longitudinal axis of the collection fiber optic.

In another embodiment, the catheter further comprises an optical element disposed about the flexible conduit, the optical element including an array of multiple facets that lie at an acute angle relative to the longitudinal axis of the flexible conduit for changing a direction of radiation transmitted along a longitudinal axis of the at least one delivery waveguide so that the radiation is emitted in a direction that is transverse to the longitudinal axis of the at least one delivery waveguide.

In another embodiment, the catheter further comprises an optical element disposed about the flexible conduit, the optical element including an array of multiple facets that lie at an acute angle relative to the longitudinal axis of the flexible conduit for changing a direction of radiation transmitted into the transmission input of the at least one collection waveguides so that the radiation is transmitted along longitudinal axes of the collection waveguides.

In another embodiment, the distal ends of the at least one collection waveguide in the region of the transmission input lie along a helical path about the longitudinal axis of the conduit.

In another embodiment, distal ends of the at least one delivery waveguide in the region of the transmission output lie along a helical path about the longitudinal axis of the conduit.

In another embodiment, the transmission output of the at least one delivery waveguide and the transmission input of the at least one collection waveguide are spaced apart at a predetermined distance in a longitudinal direction along the longitudinal axis of the conduit.

In another embodiment, the balloon comprises a polymer material that is substantially transparent to radiation at the wavelength in the range of about 250 to 2500 nanometers.

In another embodiment, the polymer material is selected from the group of materials consisting of nylon and polyethylene.

In another embodiment, the at least one delivery waveguide comprises a plurality of delivery waveguides and wherein the at least one collection waveguide comprises a plurality of collection waveguides.

In another embodiment, the at least one delivery waveguide comprises two, three or four delivery waveguides and wherein the at least one collection waveguide comprises two, three or four collection waveguides.

In another embodiment, the plurality of transmission outputs of the plurality of delivery waveguides are arranged to illuminate an interior wall of a lumen about a 360 degree portion thereof through the balloon, when the balloon is inflated within the lumen, and wherein the plurality of transmission inputs of the plurality of collection waveguides are arranged to receive radiation from the interior wall of the lumen about the illuminated 360 degree portion thereof through the balloon.

In another embodiment, the at least one delivery waveguide comprises first and second delivery waveguides and wherein the at least one collection waveguide comprises first and second collection waveguides, and wherein the transmission outputs of the first and second delivery waveguides are positioned circumferentially opposite each other relative to the flexible conduit and wherein the transmission inputs of the first and second collection waveguides are positioned circumferentially opposite each other relative to the flexible conduit, so that four quadrants of a 360 degree portion of an interior wall of the lumen can be illuminated by the radiation through the balloon and so that reflected radiation can be received from the four quadrants of the interior wall through the balloon.

In another embodiment, the transmission output of the at least one delivery waveguide comprises an uncladded fiber core sealed within a covering that is substantially transparent to radiation at the wavelength in the range of about 250 to 2500 nanometers.

In another embodiment, the substantially transparent covering comprises a cylindrical capsule containing a material having an index of refraction so as to provide an interface between the uncladded fiber core and the material in the capsule to direct incident radiation in a predetermined direction.

In another embodiment, the transmission output of the at least one delivery waveguide comprises scattering particles and a reflective terminating member so as to direct radiation in a direction that is transverse to a longitudinal axis of the at least one delivery waveguide.

In another embodiment, the balloon is sealed to the flexible conduit at a first longitudinal position and the second longitudinal position of the flexible conduit.

In another embodiment, the balloon is coupled to the conduit at a first longitudinal position of the conduit at a first portion of the balloon and wherein the balloon is coupled to the conduit at a second longitudinal position of the conduit at a second portion of the balloon, and wherein the transmission output of the at least one delivery waveguide and the transmission input of the at least one collection waveguide are located within the balloon between the first and second longitudinal positions of the conduit.

In another embodiment, the catheter further comprises a guidewire sheath coupled to the conduit at the distal end of the conduit, wherein the balloon is coupled to the guidewire sheath and conduit at a first portion of the balloon and wherein the balloon is coupled to the guidewire sheath at a second portion of the balloon.

In another embodiment, the flexible conduit comprises a core tube including a guidewire lumen.

In another embodiment, the at least one collection waveguide and the at least one delivery waveguide are positioned within a fluid transfer lumen of the core tube along a majority of its length.

In another embodiment, the at least one collection waveguide and the at least one delivery waveguide are positioned within a catheter sheath surrounding the core tube along a majority of its length.

In another embodiment, at least one of the at least one delivery waveguide and the at least one collection waveguide comprises graded-index optical fiber.

In another embodiment, at least one of the at least one delivery waveguide and the at least one collection waveguide has a numerical aperture between approximately .22 and .4.

In another embodiment, the at least one delivery waveguide comprises a fiber having a fiber core diameter of between about 9 and 100 microns.

In another embodiment, the at least one collection waveguide comprises a fiber having a fiber core diameter of between about 50 and 200 microns.

In another embodiment, the at least one delivery waveguide comprises a fiber having a fiber core diameter of about 50 microns and wherein the at least one collection waveguide comprises a fiber having a fiber core diameter of about 100 microns.

In another embodiment, a maximum outer diameter of the catheter including the flexible conduit, the at least one delivery waveguide, the at least one collection waveguide and the balloon is less than about 1.5 millimeters when the balloon is uninflated.

In another aspect, a system for probing and treating a body lumen comprises: a flexible conduit that is elongated along a longitudinal axis suitable for insertion into a body lumen, the conduit having a proximal end and a distal end; at least one delivery waveguide and at least one collection waveguide integrated with the flexible conduit; at least one radiation source connected to a transmission input of the at least one delivery waveguide, the radiation source constructed and arranged to provide radiation at a wavelength in a range of about 250 to 2500 nanometers; at least one optical detector connected to a transmission output of the at least one collection waveguide; and a lumen-expanding inflatable balloon disposed about a portion of the conduit, a transmission output of the at least one delivery waveguide and a transmission input of the at least one collection waveguide located within the balloon.

In one embodiment, the transmission output of the at least one collection waveguide is connected to a spectrometer, the spectrometer constructed and arranged to scan radiation and perform spectroscopy at the wavelength in the range of about 250 nm to 2500 nm.

In another embodiment, the spectrometer is configured to perform spectroscopy selected from the group of spectroscopy methods consisting of fluorescence, light scatter, optical coherence reflectometry, optical coherence tomography, speckle correlometry, Raman, and diffuse reflectance spectroscopy.

In another embodiment, the spectrometer is constructed and arranged to scan radiation and perform spectroscopy at a wavelength within the range of about 750 nm to 2500 nm.

In another embodiment, the spectrometer is constructed and arranged to scan radiation and perform spectroscopy using one or more ranges of wavelengths.

In another embodiment, a scan using the one or more ranges of wavelengths includes a scan using one or more discrete wavelengths.

In another embodiment, the system further comprises a controller that is programmed to automate control of activation and deactivation of the at least one radiation sources and the at least one optical detectors, to further control analysis of data collected by the system.

In another embodiment, the system is constructed and arranged for use in a medical care facility including a hospital or outpatient unit.

In another embodiment, the controller is programmed to operate a human-interactive interface that provides an operator with feedback about data and analysis of the spectroscopy, the interface providing information for real-time diagnosis.

In another embodiment, the controller is programmed to identify one or more characteristics of targeted tissue including at least one of: presence of chemical components, tissue morphological structures, water content, blood content, temperature, pH, and color.

In another embodiment, the controller is further programmed to discriminate between tissue characteristics and non-relevant artifacts including elements of the catheter and other elements artificially introduced into the body lumen.

In another embodiment, the artificially introduced elements include at least one of stents and the coatings of stents.

In another embodiment, the system further comprises a switch coupled between the at least one radiation source and the at least one delivery waveguide that selects between multiple radiation sources for application of radiation to the at least one delivery waveguide.

In another embodiment, the system further comprises a switch coupled between the at least one radiation source and the at least one delivery waveguide that selectively applies the at least one radiation source to the at least one delivery waveguide.

In another embodiment, the system further comprises a therapy delivery subsystem.

In another embodiment, the therapy delivery subsystem further comprises a tube associated with the flexible conduit through which at least one of treatment drugs and agents can be delivered.

In another embodiment, the one or more radiation sources are configured to produce an output power of the radiation of less than about 20 milliwatts at locations outside the balloon when inflated.

In another aspect, a catheter for placement within a body lumen comprises: a flexible conduit that is elongated along a longitudinal axis, the flexible conduit having a proximal end and a distal end; at least one delivery waveguide and at least one collection waveguide positioned along the flexible conduit; and a lumen-expanding inflatable balloon disposed about a portion of the conduit, a transmission output of the at least one delivery waveguide and a transmission input of the at least one collection waveguide located within the balloon, wherein the maximum outer diameter of the catheter, including the flexible conduit, the at least one delivery waveguide, the at least one collection waveguide and the balloon is less than about 1.5 millimeters when the balloon is uninflated.

In one embodiment, the at least one delivery waveguide and the at least one collection waveguide are constructed and arranged to transmit radiation at a wavelength in a range of about 250 to 2500 nanometers

In another embodiment, at least one of the at least one delivery waveguide and the at least one collection waveguide comprises graded-index optical fiber.

In another embodiment, at least one of the at least one delivery waveguide and the at least one collection waveguide has a numerical aperture between approximately .22 and .4.

In another embodiment, the at least one delivery waveguide comprises a fiber having a fiber core diameter of between about 9 and 100 microns.

In another embodiment, the at least one collection waveguide comprises a fiber having a fiber core diameter of between about 50 and 200 microns.

In another embodiment, the at least one delivery waveguide comprises a fiber having a fiber core diameter of about 50 microns and wherein the at least one collection waveguide comprises a fiber having a fiber core diameter of about 100 microns.

In another aspect, a method for providing analysis and treatment of a body lumen comprises: inserting into a body lumen a catheter including a flexible conduit, a lumen-expanding balloon, at least one delivery waveguide and at least one collection waveguide, a transmission output of the at least one delivery waveguide and a transmission input of the at least one collection waveguide being located within the balloon; maneuvering the conduit into a designated region of the body lumen designated for treatment or analysis; expanding the balloon in the designated region of the body lumen; executing spectroscopic analysis of the designated region of the body lumen using radiation at a wavelength in a range of about 250 to 2500 nanometers by radiating the designated region of the body lumen with the radiation that is supplied at the transmission output of the at least one delivery waveguide, the supplied radiation passing through the balloon where it is incident on the designated region of the body lumen, and wherein radiation is returned through the balloon to the transmission input of the at least one collection waveguide.

In one embodiment, expanding the balloon therapeutically expands the body lumen.

In another embodiment, expanding the balloon to therapeutically expand the body lumen dilates the body lumen in the designated region.

In another embodiment, executing spectroscopic analysis is performed while the balloon is expanded.

In another embodiment, the insertion and maneuvering of the conduit and the expansion of the balloon follow procedures in accordance with percutaneous transluminal angioplasty.

In another embodiment, the insertion and maneuvering of the conduit and the expansion of the balloon follow procedures in accordance with percutaneous coronary transluminal angioplasty.

In another embodiment, the balloon is expanded such that the flow of blood between the balloon and the surrounding lumen tissue is substantially stopped.

In another embodiment, the spectroscopic analysis includes the characterization of one or more pathophysiologic or morphologic factors of surrounding tissue within an endovascular region.

In another embodiment, the pathophysiologic or morphologic factors include characterizing the presence, volume, and positioning of plaque within the endovascular region.

In another embodiment, the pathophysiologic or morphologic factors further include characteristics of plaque including at least one of collagen content, lipid content, calcium content, inflammation, or the relative positioning of pathophysiologic conditions within the plaque.

In another embodiment, the method further comprises providing a stent on the lumen-expanding balloon for delivery in the designated region at the time of expanding the balloon.

In another embodiment, executing spectroscopic analysis further comprises: collecting analysis data based on the radiation that is returned through the at least one collection waveguide; and discriminating between collected analysis data associated with targeted tissue in the designated region and analysis data associated with artifacts including at least one of the balloon, a balloon expansion media, a guidewire, a stent, and an artificial material placed on a stent.

In another embodiment, the analysis data associated with artificial materials placed on stents include data associated with polymers.

In another embodiment, the analysis data associated with artificial materials placed on stents include data associated with drugs.

In another embodiment, the method further comprises determining an appropriate treatment for the designated region using the spectroscopic analysis.

In another embodiment, determining an appropriate treatment includes selecting a type of stent most appropriate for insertion.

In another embodiment, determining a type of stent most appropriate for insertion includes selecting a drug and dosage to be eluted from the stent.

In another embodiment, executing spectroscopic analysis is performed while the balloon is partially inflated.

In another embodiment, the spectroscopic analysis performed while the balloon is partially inflated is used to calculate the location of damaged tissue.

In another embodiment, the calculation of the location of damaged tissue is used to guide the position of the conduit in the lumen prior to full inflation of the balloon.

In another embodiment, the method further comprises determining a level of expansion of the balloon using the spectroscopic analysis.

In another embodiment, the spectroscopic analysis is executed on a 360 degree portion of a wall of the lumen.

In another embodiment, executing spectroscopic analysis includes selectively switching delivery of radiation between separate ones of the at least one delivery waveguides.

In another embodiment, the selective switching distributes radiation to radiate predefined quadrants about the circumference of the balloon.

In another embodiment, the selective switching comprises selective operation of multiple radiation sources.

In another embodiment, executing spectroscopic analysis includes selectively scanning across one or more ranges of wavelengths.

In another embodiment, executing spectroscopic analysis includes scanning using one or more ranges of wavelengths between about 750 nm and 2500 nm.

In another embodiment, the one or more ranges of wavelengths are selected from ranges of approximately 250-930 nanometers, 1100-1385 nanometers, 1600-1850 nanometers, and 2100-2500 nanometers.

In another embodiment, selectively scanning across one or more ranges of wavelengths includes scanning using one or more discrete wavelengths.

In another embodiment, expanding the balloon comprises expanding the balloon with a biocompatible liquid that substantially minimizes the effects of scattering, distortion, and deflection of the radiation.

In another embodiment, the biocompatible liquid is at least one selected from the group consisting of: carbon dioxide, saline, deuterium oxide, and glycerin.

In another embodiment, the biocompatible liquid comprises super-saturated saline solution.

In another embodiment, executing spectroscopic analysis further comprises collecting analysis data based on the radiation that is received through the at least one collection waveguide.

In another embodiment, collecting analysis data occurs within a time period of less than about 1 second.

In another embodiment, the method further comprises analyzing the collected analysis data.

In another embodiment, an amount of power emitted from the lumen-expanding balloon during the spectroscopic analysis is less than about 20 milliwatts.

In another aspect, a method of forming a catheter for placement within a body lumen comprises: providing a flexible conduit that is elongated along a longitudinal axis suitable for insertion into a body lumen, the flexible conduit having a proximal end and a distal end; providing at least one delivery waveguide and at least one collection waveguide along the flexible conduit, the at least one delivery waveguide and the at least one collection waveguide constructed and arranged to transmit radiation at a wavelength in a range of about 250 to 2500 nanometers; and providing a lumen-expanding inflatable balloon about a portion of the conduit so that a transmission output of the at least one delivery waveguide and a transmission input of the at least one collection waveguide are located within the balloon.

In one embodiment, the method further comprises forming distal portions of the at least one delivery waveguide and the at least one collection waveguide in a helical arrangement by: stripping end portions of the waveguides of outer jacketing; securing unstripped portions of the waveguides; applying a heat source to the end portions of the waveguides to be helically arranged, the heat source sufficient to make malleable the end portions; and applying forces to rotate the waveguides about a core segment and to translate longitudinally the end portions of the waveguides in the direction of their secured unstripped portions.

In another embodiment, the waveguides are helically arranged at predetermined angles by applying in a predetermined manner the forces to rotate and translate longitudinally the ends of the waveguides.

In another embodiment, securing the unstripped portions of the waveguides is performed using at least one locking member disposed about the core segment and the forces for rotating and translating are applied with a rotatably and translatably movable member disposed about the core segment.

In another embodiment, the end portions of the waveguides are translated a distance ranging from about 2 microns to 2 millimeters, while the end portions of the waveguides are rotated about 30 to 360 degrees about the core segment.

In another embodiment, the heat source provides heat at about 1600 Celsius.

In another embodiment, the balloon is laser welded to the conduit.

In another embodiment, the method further comprises providing a waveguide holder for contiguously retaining the at least one delivery waveguide and at least one collection waveguide to the flexible conduit.

In another embodiment, the at least one delivery waveguide and at least one collection waveguide are assembled with said waveguide holder prior to providing said at least one delivery waveguide and the at least one collection waveguide along the flexible conduit.

In another embodiment, the method further comprises shaping the transmission output of said at least one delivery waveguide after the assembly with said waveguide holder.

In another embodiment, the method further comprises shaping the transmission input of said at least one collection waveguide after the assembly with said waveguide holder.

In another embodiment, the waveguide holder for holding the at least one delivery waveguide and at least one collection waveguide comprises a holder body having a plurality of holes.

In another embodiment, the method further comprises aligning and fixing the plurality of holes with the longitudinal axis of the flexible conduit.

In another embodiment, the method further comprises correspondingly aligning and fixing the plurality of holes with one or more reflective surfaces.

In another embodiment, said one or more reflective surfaces are disposed radially about the flexible conduit as part of a multi-faceted reflecting element.

In another embodiment, said one or more reflective surfaces comprises a cone-shaped reflecting element aligned with the longitudinal axis of the flexible conduit.

In another embodiment, the waveguide holder for holding the at least one delivery waveguide and at least one collection waveguide comprises a holder body having a plurality of grooves disposed radially about the flexible conduit.

In another aspect, a catheter for placement within a body lumen comprises: a flexible conduit that is elongated along a longitudinal axis, the flexible conduit having a proximal end and a distal end; at least one delivery waveguide and at least one collection waveguide positioned along the flexible conduit, the at least one delivery waveguide and the at least one collection waveguide constructed and arranged to transmit radiation at a wavelength in a range of about 250 to 2500 nanometers; and a lumen-expanding inflatable balloon disposed about a portion of the conduit, a transmission output of the at least one delivery waveguide and a transmission input of the at least one collection waveguide being positioned along an outer surface of the balloon.

In one embodiment, the catheter further comprises a ring that couples body portions of the at least one delivery waveguide and the at least one collection waveguide to the flexible conduit.

In another embodiment, the transmission output of the at least one delivery waveguide and a transmission input of the at least one collection waveguide are mounted on the outer surface of the balloon.

In another aspect, a catheter for placement within a body lumen comprises: a flexible conduit that is elongated along a longitudinal axis, the flexible conduit having a proximal end and a distal end; at least one delivery waveguide and at least one collection waveguide positioned along the flexible conduit, the at least one delivery waveguide and the at least one collection waveguide constructed and arranged to transmit radiation at a wavelength in a range of about 250 to 2500 nanometers; and a lumen-expanding inflatable balloon disposed about a portion of the conduit, a transmission output of the at least one delivery waveguide and a transmission input of the at least one collection waveguide being mounted to an inner surface of the balloon.

In one embodiment, the catheter further comprises a ring that couples body portions of the at least one delivery waveguide and the at least one collection waveguide to the flexible conduit.

### BRIEF DESCRIPTION OF THE DRAWINGS

The foregoing and other objects, features, and advantages of the invention will be apparent from the more particular description of preferred embodiments of the invention, as illustrated in the accompanying drawings in which like reference characters refer to the same parts throughout the different views. The drawings are not necessarily to scale, emphasis instead being placed upon illustrating the principles of the invention.
Figs. 1A and 1B are schematic block diagrams illustrating an instrument for analyzing and medically treating a lumen, according to an embodiment of the present invention.
Fig. 2A is an expanded illustrative view of the treatment end of the catheter of Fig. 1, according to an embodiment of the present invention.
Fig. 2B is a cross-sectional view of the catheter of Fig. 2A, taken along section lines I-I' of Fig. 2A.
Fig. 2C is a cross-sectional view of the catheter of Fig. 2A, taken along section lines II-II' of Fig. 2A.
Fig. 3A is an expanded illustrative view of the catheter of Figs. 2A-2C having multiple fibers arranged in a helical configuration, according to an embodiment of the present invention.
Fig. 3B is a cross-sectional view of the catheter of Fig. 3A, taken along section lines I-I' of Fig. 3A.
Figs. 3C-3F are expanded illustrative views of the catheter of Figs. 3A-3B having multiple fibers arranged in a helical configuration, according to other embodiments of the present invention.
Fig. 4 is a close-up illustrative view of a catheter embodiment including four fibers attached to a core tube surrounded by an angioplasty balloon, according to an embodiment of the present invention.
Fig. 5 is a close-up illustrative view of a catheter embodiment including four fibers attached to a shortened guidewire sheath, according to an embodiment of the present invention.
Fig. 6A is an expanded illustrative view of the catheter of Figs. 2A-2C having multiple fibers attached to a fiber holder, according to an embodiment of the present invention.
Fig. 6B is a cross-sectional view of the catheter of Fig. 6A, taken along section lines I-I' of Fig. 6A.
Fig. 6C is an illustrative view of a catheter's distal portion, in which fibers are retained in holed fiber holders, according to an embodiment of the present invention.
Fig. 6D is a cross-sectional view of the fiber holders of Fig. 6C, taken along section lines I-I' of Fig. 6C.
Fig. 7A is an expanded illustrative view of a catheter embodiment having multiple fibers attached to a fiber holder, and arranged in a helical configuration, according to an embodiment of the present invention.
Fig. 7B is a cross-sectional view of the catheter of Fig. 7A, taken along section lines I-I' of Fig. 7A.
Fig. 7C is an illustrative view of a catheter's distal portion, in which helically arranged fibers are retained in holed fiber holders, according to an embodiment of the invention.
Fig. 7D is a cross-sectional view of a fiber holder of Fig. 7C, taken along section lines I-I' of Fig. 6C.
Fig. 7E is an illustrative perspective view of the fiber holder of Figs. 7C-7D showing trace lines of holes within which helically arranged fibers can be retained.
Figs. 7F-7G are longitudinal and side-perspective illustrative views, respectively, of the fiber holder of Figs. 7C-7E.
Fig. 8A is an illustrative view of a catheter having multiple fibers attached a movable fiber holder, and surrounded by a catheter sheath, according to an embodiment of the present invention.
Fig. 8B is a cross-sectional view of the catheter of Figs. 8A-8B, taken along section lines I-I' of Fig. 8A, according to another embodiment of the present invention.
Figs. 8C-8E are expanded illustrative views of the catheter of Fig. 8A having multiple fibers attached to the fiber holder in a helical configuration, according to other embodiments of the present invention.
Fig. 9A is a schematic diagram illustrating a catheter performing optical analysis on first and second quadrants of a body lumen, in accordance with an embodiment of the present invention.
Fig. 9B is a schematic diagram illustrating a catheter performing optical analysis on third and fourth quadrants of a body lumen, in accordance with an embodiment of the present invention.
Figs. 10A and 10B are views of a catheter illustrating sample ray traces of light transmissions in accordance with an embodiment of the invention having a "side-firing" tip fiber arrangement.
Figs. 10C and 10D are views of a catheter illustrating sample ray traces of light transmissions in accordance with another embodiment of the invention having a helical fiber arrangement.
Figs. 10E through 10G are views of a catheter illustrating sample ray traces of light transmissions in accordance with another embodiment of the invention having a conical reflector.
Fig. 10H is a view of a catheter illustrating sample ray traces of light transmissions in accordance with another embodiment of the invention having a multi-faceted reflector. FIG. 10I is a close-up perspective view of an embodiment of the multi-faceted reflector of FIG. 10H.
Fig. 10J is a cross-sectional view of a catheter having six fibers, and performing optical analysis on a body lumen, according to another embodiment of the present invention.
Fig. 11A is an illustrative view of a fiber including a beveled tip ("side-fire" arrangement) having an applied metal coating and an optical window, according to an embodiment of the present invention.
Fig. 11B is a cross-sectional view of the fiber of Fig. 11A, taken along section lines I-I' of Fig. 11A.
Fig. 11C is an illustrative view of a beveled fiber tip absent cladding on the distal part, according to an embodiment of the present invention.
Fig 11D is an illustrative view of an end of a fiber including a transparent capsule, according to an embodiment of the present invention.
Fig. 12 is a perspective view of a dual-clad optical fiber that can be employed for delivery, collection, or delivery and collection of optical energy, according to another embodiment of the present invention.
Fig. 13A is an illustrative view of a balloon catheter including a delivery fiber having a diffusing head, according to another embodiment of the present invention.
Fig. 13B is a cross-sectional view of the instrument of Fig. 13A, taken along section lines I-I' of Fig. 13A.
Fig. 13C is an expanded illustrative view of the output end of the delivery fiber of Fig. 13A including a diffuser and scattering particles, according to an embodiment of the present invention.
Fig. 13D is an expanded illustrative view of the output end of the delivery fiber of Fig. 13A including a diffuser, according to another embodiment of the present invention.
Fig. 14A is an illustrative view of a balloon catheter including a delivery fiber positioned within a core tube, wherein a guidewire sheath is located on the distal end of the balloon, according to an embodiment of the present invention.
Fig. 14B is a cross-sectional view of the instrument of Fig. 14A, taken along section lines I-I' of Fig. 14A.
Fig. 14C is a cross-sectional view of the instrument of Fig. 14A, taken along section lines II-II' of Fig. 14A.
Fig. 15A is an illustrative side view of an instrument comprising a balloon catheter and a stent, according to an embodiment of the present invention.
Fig. 15B is a cross-sectional view of the instrument of Fig. 15A, taken along section lines I-I' of Fig. 15A.
Fig. 16A is a close-up illustrative view of a catheter embodiment including delivery fibers and collection fibers that are adjacent to the outside surface of the balloon, according to an embodiment of the present invention.
Fig. 16B is a cross-sectional view of the catheter of Fig. 16A, taken along section lines I-I' of Fig. 16A.
Fig. 17A is a close-up illustrative view of a catheter embodiment including delivery fibers and collection fibers that are affixed or molded to the inside surface of the balloon, according to an embodiment of the present invention.
Fig. 17B is a cross-sectional view of the catheter of Fig. 17A, taken along section lines I-I' of Fig. 17A, according to an embodiment of the present invention.
Fig. 18A is an illustrative view of a device for helically bending a fiber assembly, in accordance with an embodiment of the present invention.
Fig. 18B, 18C and 18D are cross-sectional views of the instrument of Fig. 18A, taken along section lines I-I', II-II' and III-III' respectively, of Fig. 18A.
Figs. 19A -19D are illustrative views of the sequential steps of an instrument manufacturing process, in accordance with an embodiment of the present invention.
Figs. 20A -20G are cross-sectional views illustrating the sequential steps of performing a balloon angioplasty procedure, in accordance with an embodiment of the present invention.

### DETAILED DESCRIPTION OF EMBODIMENTS OF THE INVENTION

The accompanying drawings are described below, in which example embodiments in accordance with the present invention are shown. Specific structural and functional details disclosed herein are merely representative. This invention may be embodied in many alternate forms and should not be construed as limited to example embodiments set forth herein.

Accordingly, specific embodiments are shown by way of example in the drawings. It should be understood, however, that there is no intent to limit the invention to the particular forms disclosed, but on the contrary, the invention is to cover all modifications, equivalents, and alternatives falling within the spirit and scope of the claims. Like numbers refer to like elements throughout the description of the figures.

It will be understood that, although the terms first, second, etc. may be used herein to describe various elements, these elements should not be limited by these terms. These terms are used to distinguish one element from another. For example, a first element could be termed a second element, and, similarly, a second element could be termed a first element, without departing from the scope of the present disclosure. As used herein, the term "and/or" includes any and all combinations of one or more of the associated listed items.

It will be understood that when an element is referred to as being "on," "connected to" or "coupled to" another element, it can be directly on, connected to or coupled to the other element or intervening elements may be present. In contrast, when an element is referred to as being "directly on," "directly connected to" or "directly coupled to" another element, there are no intervening elements present. Other words used to describe the relationship between elements should be interpreted in a like fashion (e.g., "between" versus "directly between," "adjacent" versus "directly adjacent," etc.).

The terminology used herein is for the purpose of describing particular embodiments and is not intended to be limiting of the invention. As used herein, the singular forms "a," "an" and "the" are intended to include the plural forms as well, unless the context clearly indicates otherwise. It will be further understood that the terms "comprise," "comprises," "comprising," "include," "includes" and/or "including," when used herein, specify the presence of stated features, integers, steps, operations, elements, and/or components, but do not preclude the presence or addition of one or more other features, integers, steps, operations, elements, components, and/or groups thereof.

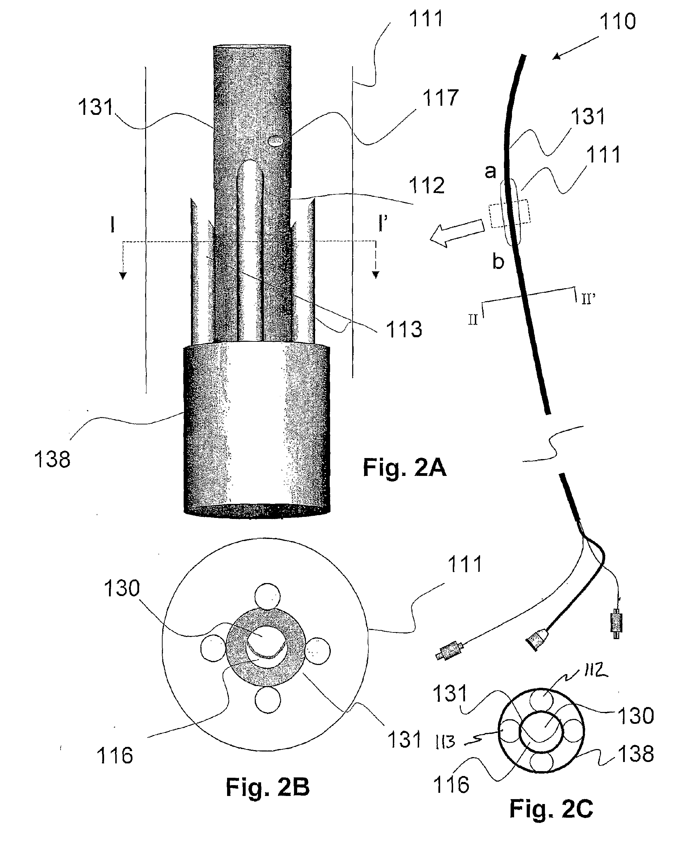

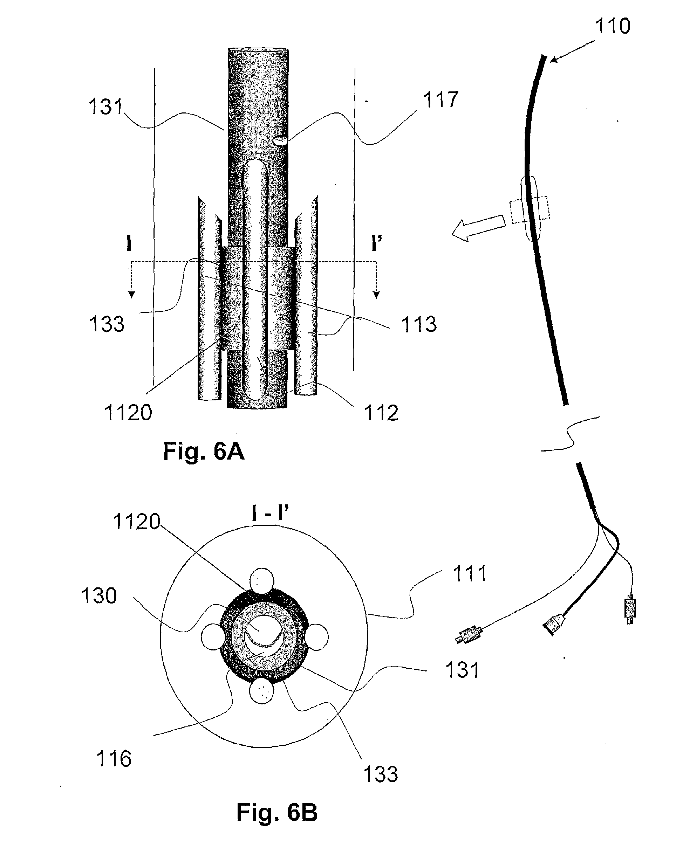

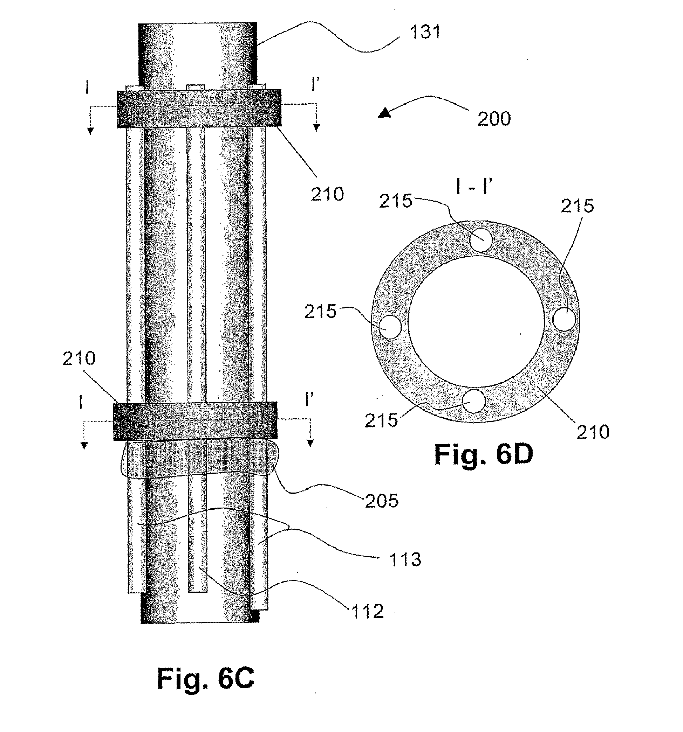

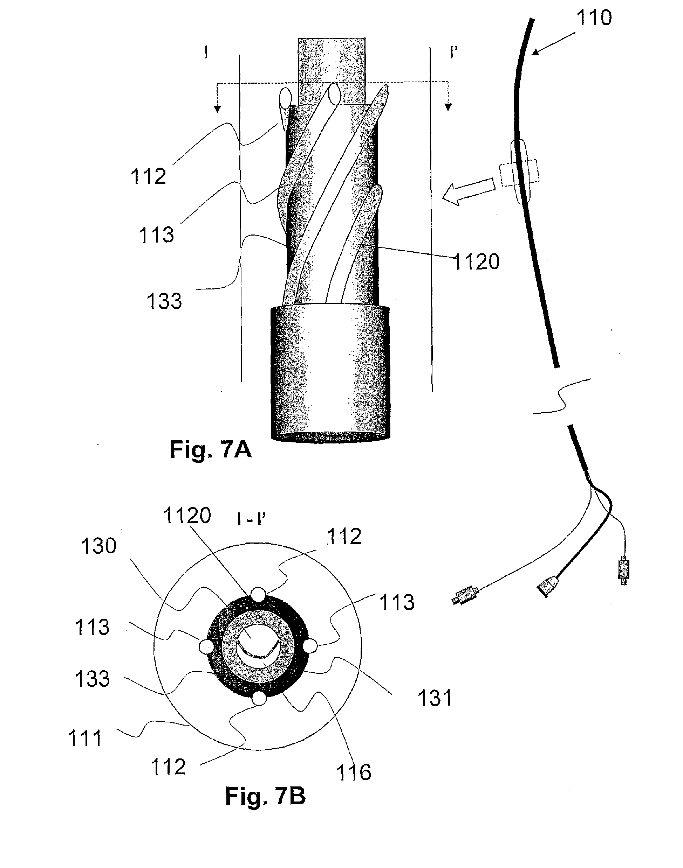

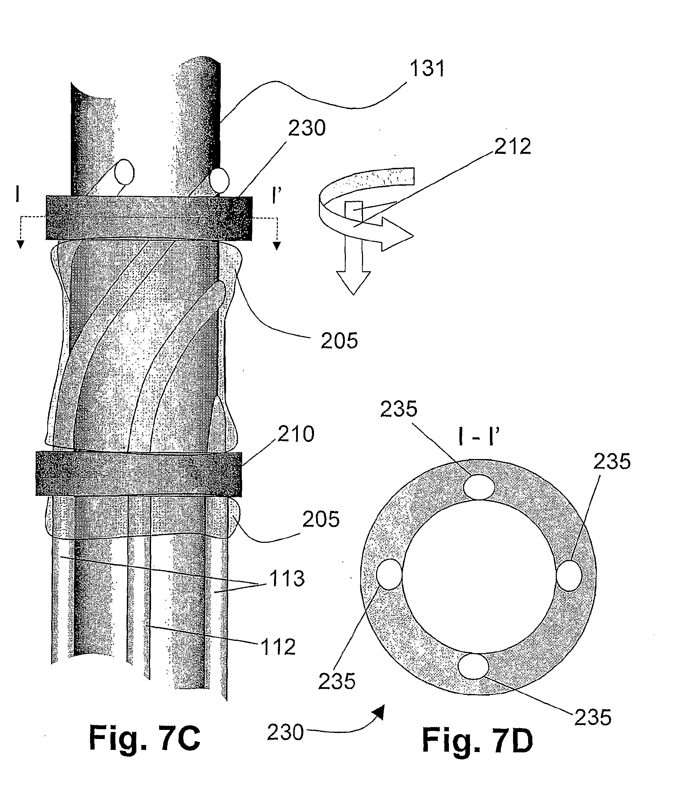

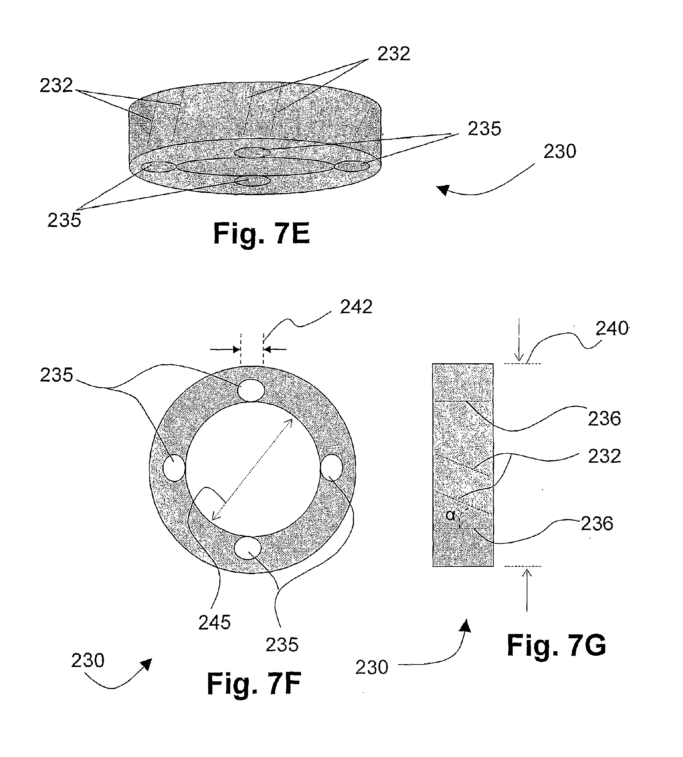

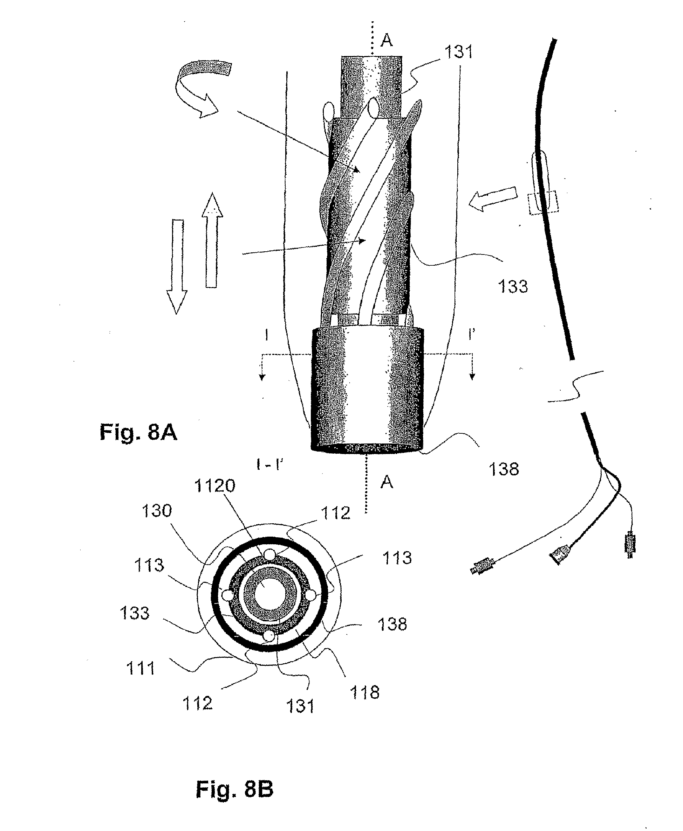

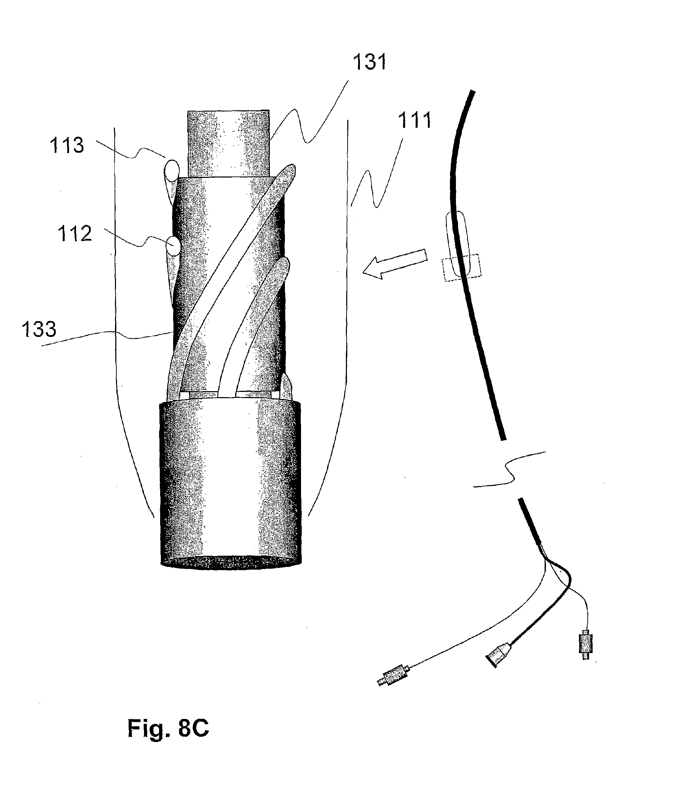

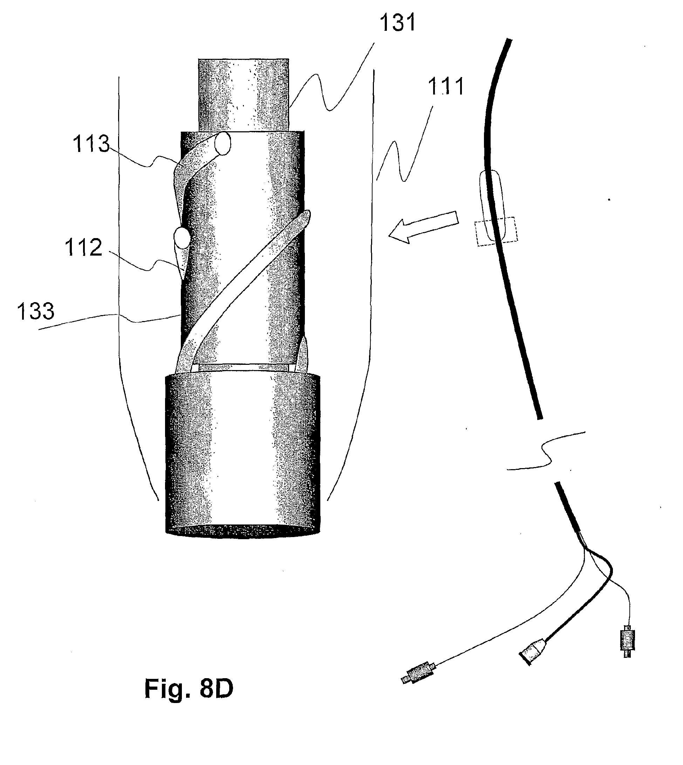

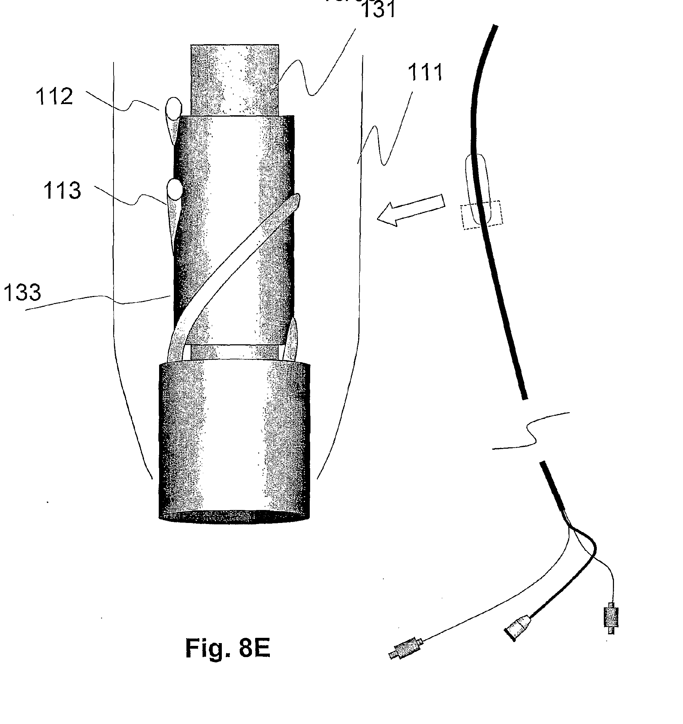

As used herein, the term "contiguously retained", when referring to the interrelationship of the delivery and collection waveguides to the flexible conduit of the catheter to which they are attached, means that they are retained in a position that is proximal, or near, the conduit. For example, the waveguides can be bonded directly to the conduit, for example as shown and described in connection with the embodiment of FIGs. 2-5 below, among others, the waveguides can be bonded to a retainer that is coupled to the conduit, for example as shown and described in connection with the embodiments of FIGs. 6 and 7 below, among others, or the waveguides can be bonded to a retainer that slides or rotates relative to the conduit, for example as shown and described in connection with the embodiments of FIGs. 8 below, among others. In each of these cases, the waveguides are physically connected to the conduit, either directly or indirectly via a fixed or moveable retainer that is in turn coupled to the conduit; the term "contiguously retained" is intended to encompass these configurations and other related configurations.

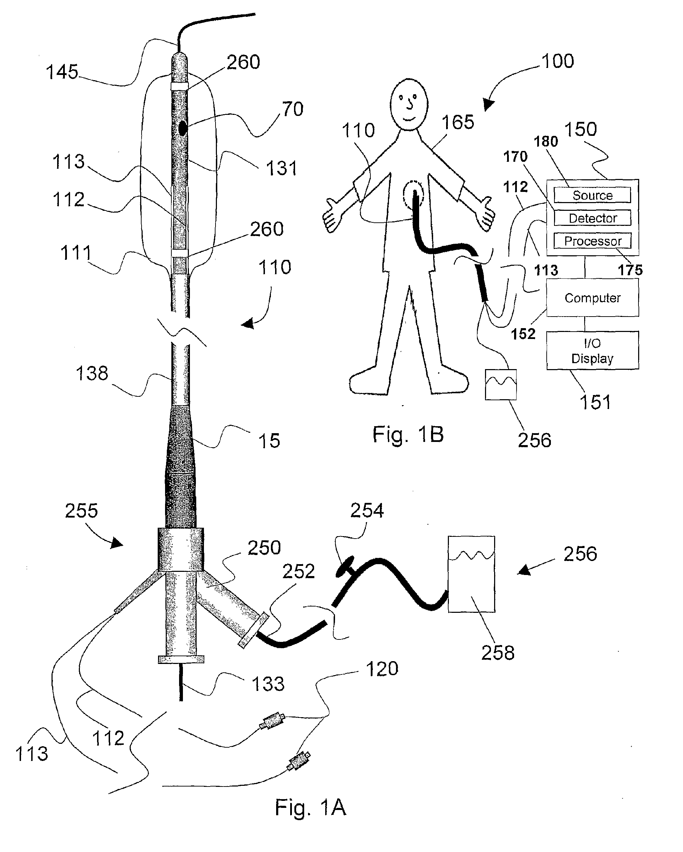

Fig. 1A is an illustrative view of a balloon catheter assembly 110 with integrated optics for analyzing ad medically treating a lumen, according to an embodiment of the present invention. The catheter assembly 110 includes a catheter sheath 138 with one or more collection fiber(s) 112 and one or more delivery fiber(s) 113, and a guidewire sheath 131 with guidewire 145. The distal end of catheter assembly 110 includes a balloon 111 within which is enclosed the delivery and collection ends of delivery fiber(s) 112 and collection fiber(s) 113, respectively. The proximate end of balloon catheter assembly 110 includes a junction 15 attaching catheter sheath 138 to a connector subassembly 255. Fibers 112 and 113 are fitted with connectors 120 (e.g. FC/PC type) compatible for use with commercially available light sources and/or analyzing devices such as a spectrometer 150 (as shown in high-level diagram Fig. 1B). Fibers 112 and 113 can alternatively be fitted with various commercially distributed multi-port connectors (not shown). Two radiopaque marker bands 260 are fixed about guidewire sheath 131 in order to allow an operator to obtain information about the location of catheter 110 in the body of a patient (e.g. with the aid of a fluoroscope).

Connector subassembly 255 includes a flushing port 250 for supplying or removing liquid/gas 258 through port 70 for expanding or contracting balloon 111. Liquid/gas 258 is held in a tank 256 from which it is pumped in or removed from balloon 111 by actuation of a knob 254. Liquid/gas 258 can alternatively be pumped with the use of automated components (e.g. switches/compressors/vacuums). Solutions for expansion of the balloon are preferably non-toxic to humans (e.g. saline solution) and are substantially translucent to the selected light radiation.

Further reference is now made to Fig. 1B, a schematic high-level block diagram illustrating an instrument 100 for the analysis and medical treatment of a body lumen according to an embodiment of the present invention. The instrument 100 comprises a catheter 110 that is constructed and arranged for insertion into a body lumen of a subject 165 undergoing treatment, for example, an artery, vein, organ, or other body cavity of the subject 165. Delivery fiber(s) 112 are connected to a light source 180 integrated into a spectrometer 150 and collection fiber(s) 113. are connected to a detector 170 (also integrated in spectrometer 150). Spectrometer 150 can process spectroscopy with the aid of a processor 175. A computer 152 connected to spectrometer 150 can provide an interface for operating the instrument 100 and to further process spectroscopic data (including, for example, through chemometric analysis) in order to diagnose and/or treat the condition of subject 165. Input/output components (I/O) and viewing components 151 are provided in order to communicate information between, for example, storage and/or network devices and the like and to allow operators to view information related to the operation of the instrument 100.

The catheter 110 can further comprise a therapy tube (not shown) that is attached to a therapy system, which can be used, for example, to treat a diseased artery. In this example, drugs can be delivered to the diseased artery via the therapy tube.

Fig. 2A is an expanded illustrative view of the treatment end of the catheter 110 of Fig. 1, according to an embodiment of the present invention. Fig. 2B is a cross-sectional view of the catheter 110 of Fig. 2A, taken along section lines I-I' of Fig. 2A and Fig. 2C is a cross-sectional view of the catheter of Fig. 2A, taken along section lines II-II' of Fig. 2A. The catheter 110 includes a balloon 111, a core tube 131 including a longitudinal guidewire lumen 130 and fluid transfer lumen 116, at least one delivery fiber 112, and at least one collection fiber 113. In one embodiment, the catheter 110 is an angioplasty balloon catheter. In this manner, when the catheter 110 is placed in a body lumen, for example, an artery, the catheter can optionally be used to perform a balloon angioplasty and/or to place a stent in the artery. In alternative embodiments, the delivery fiber 112 can comprise a single delivery fiber or multiple delivery fibers, or the collection fiber 113 can comprise a single collection fiber or multiple collection fibers.

At least one energy source 180 is attached to a proximal end of the delivery fiber 112. At least one detector 170 is attached to a proximal end of the collection fiber 113. The energy source 180 generates electromagnetic radiation, for example, optical radiation, that is transmitted from the proximal end of the delivery fiber 112 to a distal end. The distal end of the delivery fiber 112 is positioned within the balloon and emits the optical radiation at the balloon 111, whereby the radiation is directed to the target region at an inner surface of the body lumen wall. Radiation is reflected from the body lumen wall and collected at a distal end of the collection fiber at the treatment end of the catheter 110. The spectral features of the lumen wall determine the amount, and type of radiation that is reflected and/or otherwise emitted from the lumen wall. The collected radiation is captured at the distal end of the collection fiber 113 and is directed by the collection fiber 113 to the proximal end, whereby the detector 170 processes the reflected radiation as signal data.

As shown in FIG. 1B, radiation source 180 and detector 170 are connected with and/or incorporated into spectrometer system 150. Various embodiments provide a spectrometer configured to perform spectroscopic analysis within a wavelength range between about 250 and 2500 nanometers and include embodiments having ranges particularly in the near-infrared spectrum between about 750 and 2500 nanometers. Further embodiments are configured for performing spectroscopy within one or more subranges that include, for example, about 250-930 nm, about 1100-1385 nm, about 1600-1850 nm, and about 2100-2500 nm. In other embodiments, a single wavelength, a plurality of discrete wavelengths, or a range of continuous wavelengths are used for scanning during an analysis. Large scan ranges and repeated scans provide more accurate data but can unsafely increase the time of the procedure, which, in one embodiment, preferably will not exceed about 1 second. The speed of a single scan will also preferably be sufficient to minimize the effects of motion artifacts, including those from the pumping of the heart. The power output of the radiation source is preferably greater than about 10 milliwatts in order to mitigate various losses along the delivery and collection paths, including those associated with travel through the balloon material and balloon expansion media. Embodiments of spectrometers providing preferable power outputs include laser-scanning spectrometers.

Embodiments include one or more commercially available spectrometers for scanning across multiple bands. For example, a single spectrometer can be employed, such as an AXSUN Technologies, Inc. (of Billerica, MA) IntegraSpec XL (Uno) CH spectrometer that provides a complete scan range of approximately 1550 to 1800 nm at about 25 milliseconds per scan and scans about 32 times to capture a set of data in about .8 seconds. Another embodiment includes one or more StellarNet, Inc. (of Tampa, FL) EPP2000 fiber optic spectrometers which can provide scans in the wavelength range of about 190 to 1700 nm. Ocean Optics, Inc. (of Dunedin, FL) provides many user-configurable spectrometers for output in the wavelength range of between about 200 to 1100 nm.

The reflected and collected radiation includes information which can be spectroscopically analyzed to obtain certain characteristics of the lumen wall, such as a change of chemical components, tissue morphological structures, water/blood content, and physiological parameters (e.g. temperature, pH, color, intensity) on the lumen wall. A spectroscopic analysis system 150 is connected to the detector 170, for processing the signal data received by the detector 170. The processed signal data can be output to a display 151 in the form of user-readable text and graphics. In this manner, the data can be analyzed by a user, for example, a physician, in real time, if desired.

In this manner, a physician can obtain real-time information from the diseased area undergoing treatment. This information may include all of pathological and/or pathophysiologic results, which, in the conventional approaches, are needed to sample tissue and/or blood from the lumen wall and which may require up to several days to analyze the information. The present invention permits the physician to immediately select the most suitable treatment for his/her patients according to the information obtained and processed from received optical radiation.

As shown in Figs. 2A-2C, the balloon 111 is affixed to the core tube 131 about the body of the core tube 131 at or near its distal end. The balloon 111 surrounds the distal ends of the delivery fibers 112 where the optical energy is output, and the distal ends of the collection fibers 113 where the reflected optical energy is collected. In one embodiment, the balloon 111 surrounds the body of the core tube 131, and is sealed at the body of the core tube 131 or a protective sheath 138 surrounding the optical fibers 112 and 113 and the core tube, at two locations, for example, at locations a, b, such that the distal end of the core tube 131 extends beyond the balloon 111 in a longitudinal direction. The protective sheath 138 is comprised of a material that provides exceptional lubricity and biocompatibility, such as polyethylene, polypropylene, and polyurethane, or silicon. In one embodiment, the balloon 111 is sealed at locations a, b by applying heat to locations a, b, which welds the balloon to the core tube 131 or protective sheath. Alternatively, glue or adhesive, or other known techniques, can be applied for sealing the balloon 111 to the core tube 131. In this manner, during a medical procedure, a guidewire can be threaded through the body lumen to the target region in advance of the balloon catheter. The balloon 111 attached to the core tube 131 or the protective sheath can then subsequently be positioned into the target region by tracking the guidewire according to conventional angioplasty procedures.

In a preferred embodiment, the balloon 111 is an angioplasty balloon. The angioplasty balloon 111 can be inflated with a fluid that is transferred from a fluid source (not shown) through a fluid transfer lumen 116 in parallel with the guidewire lumen 130, wherein the fluid transfer lumen 116 and the guidewire lumen 130 are both surrounded by the core tube 131. The fluid is output to the balloon 111 via a port 117 that is connected to the fluid transfer lumen 116. The port 117 is located in the portion of the core tube 131 that is surrounded by the balloon 111. The port 117 and fluid transfer lumen 116 also permit the fluid to be removed from the balloon during deflation of the balloon, for example, prior to removal of the catheter from the body lumen. Directed optical radiation, for example, light, from the delivery fiber 112, and reflected optical radiation, for example, light, from the lumen wall may both be transmitted through the fluid-filled balloon during lumen wall data collection. It is therefore preferred that the properties of the fluid are such that the fluid minimizes any undesirable optical effects such as absorption, scattering, deflection, or distortion of the optical radiation that may occur as the optical radiation passes through the balloon 111 to/from the lumen wall. In this manner, the fluid that fills the balloon 111 can be either a liquid or gas, and preferably comprises saline, deuterium oxide, glycerin, or other liquids or gases that minimize the abovementioned optical effects.

During a balloon angioplasty therapy treatment, which can be performed in conjunction with the optical lumen wall analysis procedure described herein, the angioplasty balloon 111 is inflated with the fluid supplied via the port 117 at a sufficient pressure until the balloon 111 is sufficiently expanded against the stenosis region for treatment. At this time, the pressure of the balloon 111 against the lumen wall is preferably sufficient to obstruct blood flow. This feature is preferable for optical radiation collection from a blood vessel wall, since the balloon 111 is in direct contact with the wall. It is preferred that no blood is interposed between the balloon 111 and the lumen wall that may cause optical loss such as absorbance to occur, or other undesirable optical effects. Since there is little or no blood between the balloon 111 and the lumen wall, spectral features of the lumen wall 160 can therefore be measured with a high degree of accuracy. The embodiments of the present disclosure are also advantageous over conventional balloon catheters, which can only be used for treatment purposes. In contrast, the disclosed embodiments allow for simultaneously performing both optical analysis and angioplasty treatment, such as an angioplasty procedure or stent insertion, to gain the benefit of both treatment and diagnosis, wherein both spectroscopic analysis and angioplasty treatment can be performed in the same procedure, by the same catheter, without the need for removing and inserting different catheters for the two different purposes.

Fiber construction and size can be selected based on parameters relating to the type of analysis being performed, the number and sizes of discrete regions being analyzed, space, strength, and flexibility constraints and/or cost constraints. In various embodiments, fibers may be constructed of different materials and thicknesses of core, cladding, and jackets. Fibers may also be constructed of graded-index cores in order to increase the numerical aperture and power while retaining small core diameters. Embodiments of the invention include graded index fibers of numerical apertures between approximately .22 and .4. Embodiments include the use of delivery waveguides with core diameters between about 9 and 100 microns and the use of collection waveguides with a fiber core between about 50 and 200 microns. Lucent Technologies Specialty Fiber Group, for example, provides fibers having core diameters between about 62.5 µm to 1500 µm and numerical apertures between about 0.11 to 0.48. Yangtze Optical Fiber and Cable Co., Ltd. of Wuhan, China (See http://yofcfiber.com) provides single-mode fiber cores with diameters as small as about 9 µm.

In one embodiment, two delivery fibers and two collection fibers are included, wherein the delivery fibers have a numerical aperture of approximately .31, a graded core diameter of approximately 50 micrometers, a cladding layer thickness of approximately 9 to 10 micrometers, and a jacket of approximately 4 to 5 micrometers. Corresponding collection fibers can, for example, be graded indexed with a core numerical aperture of about .22, a core diameter of approximately 100 micrometers, a cladding layer thickness of approximately 10 micrometers, and a jacket thickness of approximately 10 micrometers. Smaller sized fibers with relatively high numerical apertures (NAs) (e.g. between about .22 and .4) allow for embodiments of a catheter system in accordance with the invention which have maximum outer diameters of about 1.5 mm or less, assuming an uninflated balloon that can provide smooth deployment within the cardiovascular system.

In one embodiment, the collection fibers 113 receive the reflected optical radiation from the lumen wall through the balloon 111 in a transverse direction relative to the longitudinal axis of the core tube 131, and direct the reflected optical radiation in a longitudinal direction from the distal end of the collection fibers 113 to the proximal end of the collection fibers 113, whereby the received optical radiation is transmitted to a detector 170. The collection fibers 113, like the delivery fibers 112, can comprise optical fibers including a core, a doped cladding, and a protective jacket. The optical radiation is received at the distal end of each collection fiber 113, and is directed from the distal end to the proximal end of the collection fiber 113. The term "transverse" as used herein for example when referring to a direction of emission or collection of radiation relative to a longitudinal axis of a fiber core or conduit includes all angles, whether acute, obtuse, or perpendicular, other than parallel to the longitudinal axis of the fiber or conduit.

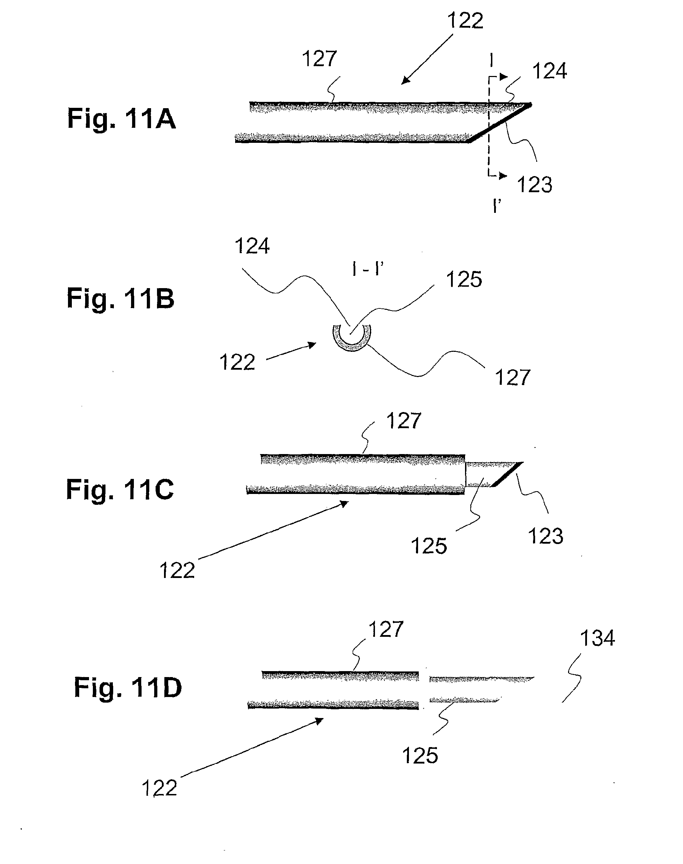

The collection fibers 113 can be arranged to receive optical radiation at the treatment region much in the same manner as the delivery fibers 112 transmit radiation in the treatment region, but in an opposite direction. For example, in one embodiment, the collection fibers 113 extend along longitudinal axes, and are arranged in parallel with, and adjacent to, the delivery fibers 112 and the core tube 131. The distal end of each collection fiber 113 receives reflected optical radiation in a similar manner as delivery fibers 112 distribute radiation, whereby the reflected optical radiation impinges on an optical component at the distal end of the collection fiber 113. Optical components can include lenses, mirrors or optical reflectors. The optical components can optionally be integrated into the respective distal ends of each collection fiber 113 and/or delivery fiber 112 such as, for example, in accordance with the "side-fire" arrangement described further in reference to Fig. 11A. The optical components may also be external to the collection fibers 113, including those in sufficient proximity to collection fiber tips to receive the reflected optical radiation from the lumen wall 160. In one embodiment, the collection fibers 113 are attached to grooves in a fiber holder or core tube, the grooves formed to be parallel to each other and longitudinally rotated along a majority of the body of the core tube and along a helical path at the distal end of the treatment region. In this manner, the collection fibers 113 conform to the grooves such that the reflected optical radiation travels along the helical path from the proximal end to the distal end of each collection fiber 113.

The balloon 111 can be composed of a material such as nylon, or other translucent polymers. In one example, balloon 111 comprises, for example, a thin, optically clear, polyethylene balloon. In embodiments where the optical radiation is directed through the surface of the balloon, it is preferred that the surface of the balloon be sufficiently transparent or translucent to permit a maximum amount of directed and reflected optical radiation to be transmitted through the balloon surface, while minimizing any reflectance or loss.

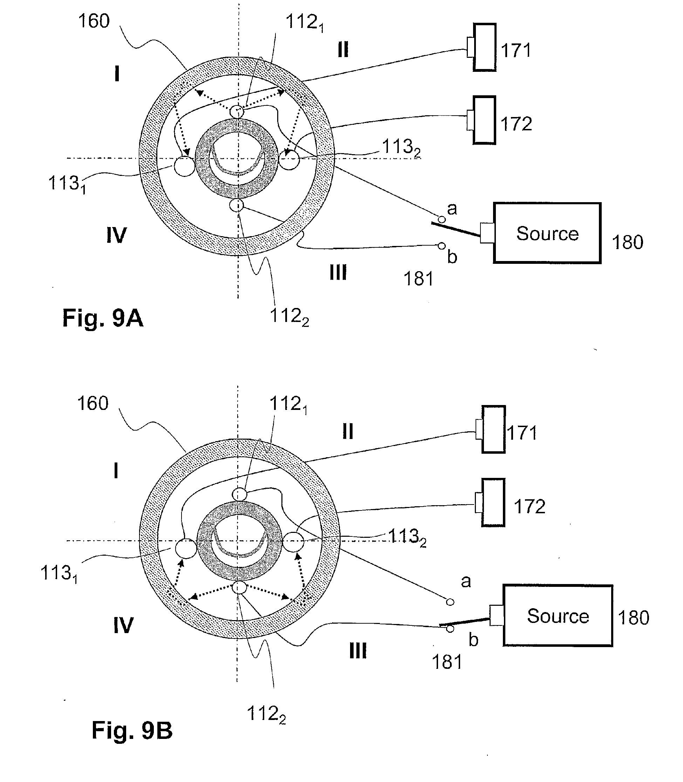

Returning to Figs. 1A and 1B, and also referring to Figs. 9A and 9B, the source 180 is attached to the proximal end of the delivery fiber 112, and generates radiation that is transmitted by the delivery fiber 112 to a surface of the lumen wall to be analyzed. The source 180 comprises, for example, an electromagnetic radiation source that emits, for example, optical radiation. The optical radiation can be delivered by the source 180 to multiple delivery fibers, for example, using one or more optical switches. One source 180 can be shared by more than two delivery fibers 112₁ and 112₂ as shown in Figs. 9A and 9B. As shown in Fig. 9A, the source 180 is made to illuminate quadrants I and II of the body lumen wall 160 via the delivery fiber 112₁, when the optical switch 181 is at position a. The collection fibers 113₁ and 113₂ receive the optical radiation from quadrant I and II, respectively. As shown in Fig. 9B, when the optical switch 181 is at position b, the source 180 illuminates quadrants III and IV, instead of quadrants I and II, via the delivery fiber 112₂. The optical switch can be made to toggle between position a and position b under control of a programmable controller or computer (not shown). The collection fibers 113₁ and 113₂ then receive the optical radiation from quadrants III and IV, instead of quadrants I and II, as shown in Fig. 9B. This shared-source configuration can therefore reduce the number of the sources, delivery and collection fibers, and detectors when collecting data from multiple areas of a lumen. Alternatively, multiple sources 180 can be used to deliver radiation to multiple delivery fibers 112. It is preferred that the source 180 generates energy at a wavelength or wavelengths in the near-infrared range from about 750 nm to about 2500 nm. Source 180 may also optionally provide radiation in the visible range, including wavelengths of about 100 nm to about 750 nm.

During operation, each delivery fiber 112 transmits optical radiation output by the source 180 from the proximal end to the distal end of the delivery fiber 112. At the distal end of the fiber, the optical radiation is then directed to a target region on the lumen wall. The radiation propagates from the distal end of the delivery fiber 112 through the fluid of the interior of the balloon 111, and through the surface of the balloon 111 that is in contact with the lumen wall, and is incident on the target region of the lumen wall.

The, optical radiation is directed from the proximal end of each delivery fiber 112 in an axial and/or a radial direction to the target region, in accordance with one or more embodiments disclosed in the present specification. In one embodiment, the delivery fibers 112 are positioned along a longitudinal axis of, and parallel to, the core tube 131 and the balloon 111. The emitted optical radiation impinges on an optical component attached to an angled fiber tip at the distal end of each delivery fiber 112. Such optical components can include mirrors or optical reflectors that are integrated into the respective distal ends of the delivery fibers 112. Alternatively, the optical components may be external to the delivery fibers 112, but in relative proximity to the distal ends of the delivery fibers 112 to permit the optical radiation to transversely, radially, or axially exit the catheter 110. The optical components external to the fibers may be controlled by the spectroscopic analysis system 150, for example, to change the reflected surface angle of the mirrors or reflectors. Alternatively, the emitted and collected radiation can be transmitted directly from, and received directly by, the delivery and collection fibers 112, 113, in accordance with embodiments described in detail herein.

In the example embodiments described herein, optical radiation is transmitted through the balloon 111, and impinges on a target region at the lumen wall to be analyzed, the lumen wall abutting the outer surface of the balloon 111. Also, in the embodiments described herein, the reflective surface of a cleaved or polished fiber, mirror, or optical reflector can be adjusted or shaped to deliver a wider or narrower beam of optical radiation to the target area, thereby increasing or decreasing the area of radiation. In addition, multiple delivery fibers can be spaced accordingly to each direct a beam of optical radiation at a specific target region, wherein the multiple beams of optical radiation impinge multiple target regions. The target region, for example, a diseased area, can be partitioned into the more specific regions, whereby additional detailed information about the smaller diseased area can be obtained. Here, each of the beams passes through the surface of the balloon and impinges a respective section of the target region, for example, one or more quadrants of the target region. Alternatively, each fiber having a respective reflective surface can be configured to permit multiple beams of optical radiation of the delivery fibers to overlap or intersect each other, and thereby impinge a single target region.

The reflected radiation is collected at a distal end of each collection fiber 113 and transmitted to the proximal end to a detector 170. The detector 170 generates highly accurate signals from the received radiation. The spectroscopic analysis system 150 receives the signals from the detectors, and processes the signals, resulting in data that can be used by a system operator, for example, to determine lumen properties such as an amount of, or type of, plaque on the vascular wall. The spectroscopic analysis system 150 is attached to the proximal end of the collection fibers, and outputs the processed signal data to a display 151 in the form of user-readable text and graphics.

The spectroscopic analysis system 150 can perform conventional spectroscopy, for example, Raman spectroscopy, by using a commercially available spectrometer. Alternatively, infrared and near-infrared spectroscopy, fluorescence spectroscopy, optical coherence reflectometery, optical coherence tomography, or diffuse-reflective, near-infrared spectroscopy may be performed. In addition, the spectroscopic analysis system 150 can optionally perform control and management functions of various elements of the instrument 100, such as the source 180 and detector 170. For example, the spectroscopic analysis system 150 can control the source 180 to generate a beam of optical radiation at a given wavelength or range of wavelengths and/or at a required power. In another example, the spectroscopic analysis system 150 can adjust the angle of mirrors and reflectors that are integrated with the fiber tips or provided external to the fiber tips. This permits the area of radiation on the balloon surface to be changed, and to thereby increase or decrease the area of target region.

In one embodiment, prior to a lumen wall analysis, the catheter 110 can be made to collect data, and the spectroscopic analysis system 150 can be made to process the data and discriminate between relevant data for making a diagnosis, such as data from targeted tissue, and other data not pertinent for making diagnosis including, for example, data on the spectral features of the balloon 111. Such features may include, for example, the spectral strength of the balloon, or an area of expansion of an inflated balloon, or guidewire and/or stent "shadows" or spectral strength of the fluid in the balloon 111. These spectral features pose a risk of interfering with received radiation, but this risk can be mitigated or eliminated by a software program in a data analysis procedure via the spectroscopic analysis system 150 that compensates for such features.

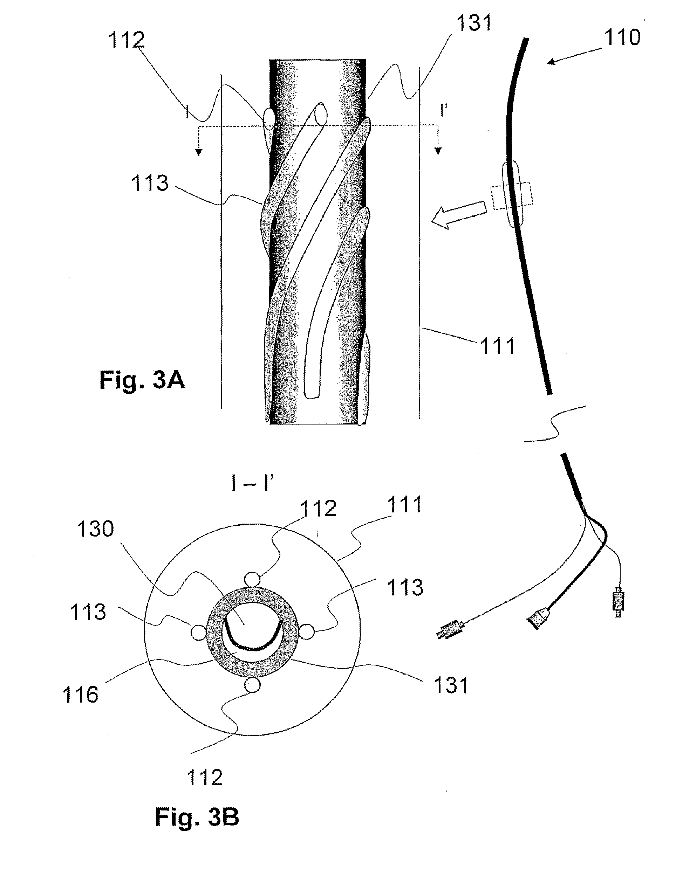

Figs. 3A-3F are expanded illustrative views of a catheter having multiple fibers arranged in a helical configuration, according to embodiments of the present invention. In Figs. 3A-3F, delivery fibers 112 and collection fibers 113 are attached to the core tube 131 along a helical path, and are spaced apart from each other at equal distances, such that the fibers attached to the core tube 131 are parallel to each other. In this manner, the fiber tips are angled with respect to the axis of the core tube 131 due to helical configuration of the fibers. This angle can range from 0 degrees (i.e., parallel to the axis of the core tube 131) to 90 degrees (i.e., perpendicular to the axis of the core tube 131), depending on the angle of the helical bend of the fibers. The optical radiation is directed from the delivery fibers 112 directly to the target region of the lumen wall at an angle. In this manner, optical radiation can be received by the angled collection fibers 113 from the target region at the lumen wall directly, without the need for an additional reflector. The respective radiation angles from the delivery fibers 112 and the collection fibers 113 may be the same, or different.

Fig. 3A is an expanded illustrative view of the catheter 110 of Fig. 2A having multiple fibers arranged in a helical configuration, according to an embodiment of the present invention. Fig. 3B is a cross-sectional view of the catheter of Fig. 3A, taken along section lines I-I' of Fig. 3A. In this embodiment, four fibers are employed, for example, comprising two delivery fibers and two collection fibers. Any number of fibers of two by *n* times (2*n*) can be arranged in a helical configuration to deliver and receive optical radiation. The ***n*** herein is an integer at least equal to 1 (1, 2, 3, etc.). The *n* delivery fibers can deliver optical radiation, and the *n* collection fibers can receive optical radiation. In this manner, the additional delivery fibers permit a larger target region or multiple target regions to receive the delivered optical radiation, and the additional collection fibers receive a greater window of reflected optical radiation from the target region or regions. In other embodiments, an odd number of delivery or collection fibers can be used. The numbers of delivery and collection fibers can be the same, or different, depending on the desired application.

In the example embodiments of Figs. 3A-3B, two delivery fibers 112 and two collection fibers 113 are attached to the outer surface of the core tube 131, for example, using glue or adhesive. The fibers 112, 113 are formed along helical paths about the core tube 131. The helical paths are formed at angles ranging from 30-180 degrees of rotation about the core tube 131. In this manner, the delivery fibers 112 can direct optical radiation to a target region at any location along the lumen wall in a 360 degree radius. For example, a portion of the lumen wall having a 360 degree radius can be partitioned into 90 degree sections, or quadrants. As such, the delivery fibers 112 and collection fibers 113 can collectively permit data to be acquired from any quadrant of the lumen wall.

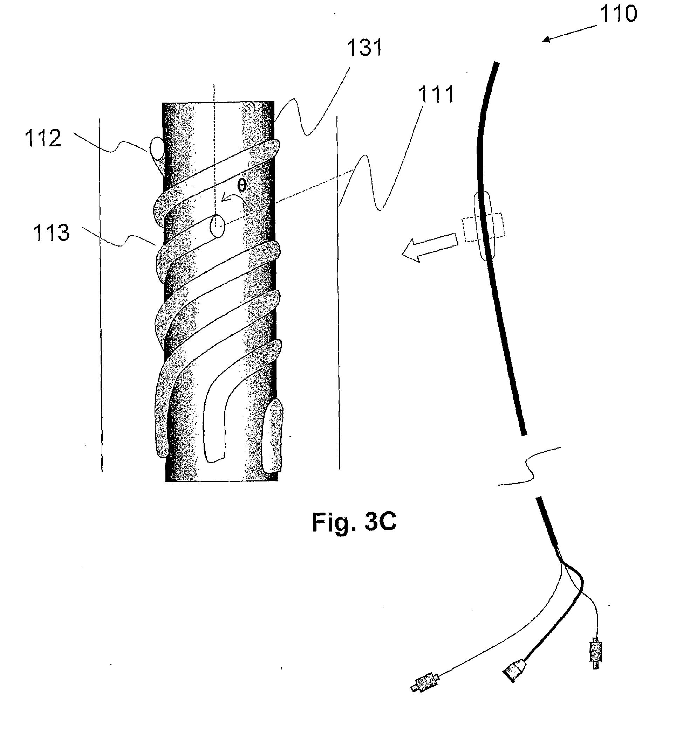

Fig. 3C is an expanded illustrative view of the catheter 110 of Fig. 3A having four fibers arranged in a helical configuration, wherein the fibers are formed to angularly extend about the core tube along a helix, according to an embodiment of the present invention. Specifically, the two delivery fibers 112 and two collection fibers 113 are attached to the outer surface of the core tube 131, and undergo angular rotation about the core tube 131. A maximum light reflection angle ? can be achieved of up to about 90 degrees in this example, assuming a flat-faced fiber output. The larger light reflection angle results in a shorter pathway of optical radiation of both delivery and collection channels in fluid-filled balloon 111, whereby energy loss of optical radiation in the fluid-filled balloon 111 is reduced.

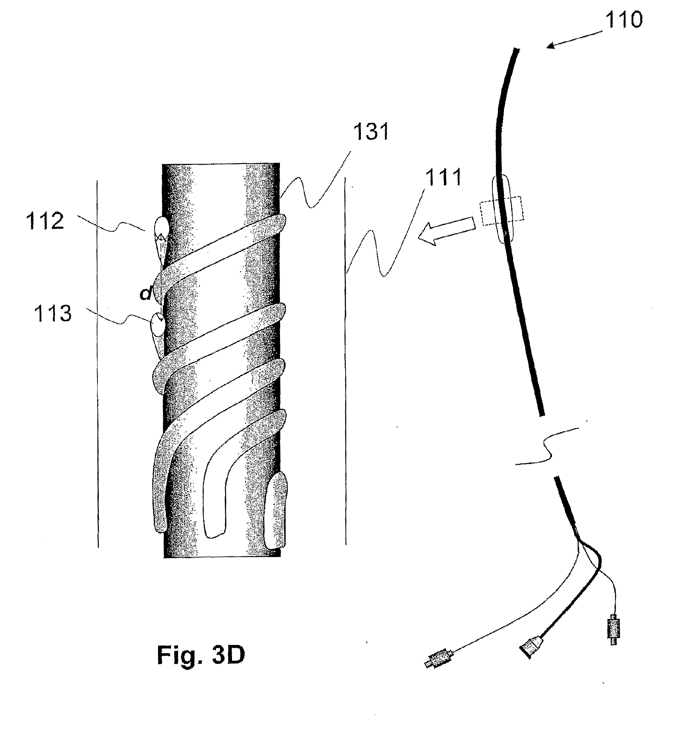

Fig. 3D is an expanded illustrative view of the catheter 110 of Fig. 3A having four fibers arranged in a helical configuration, wherein the fibers are formed to be arranged in a helix, according to an embodiment of the present invention. Specifically, the fibers 112, 113 are attached to the outer surface of the core tube 131, and undergo angular rotation about the core tube 131. The helical paths are spaced and positioned such that a desired separation distance ***d*** along the longitudinal axis of the core tube 131 between the tips of the delivery fibers 112 and collection fiber 113s can be obtained. The fiber separation ***d*** is important to determine from which layer(s) of the target wall data is collected. For example, a wider fiber separation ***d*** permits data to be collected within the deeper layers of the target wall.

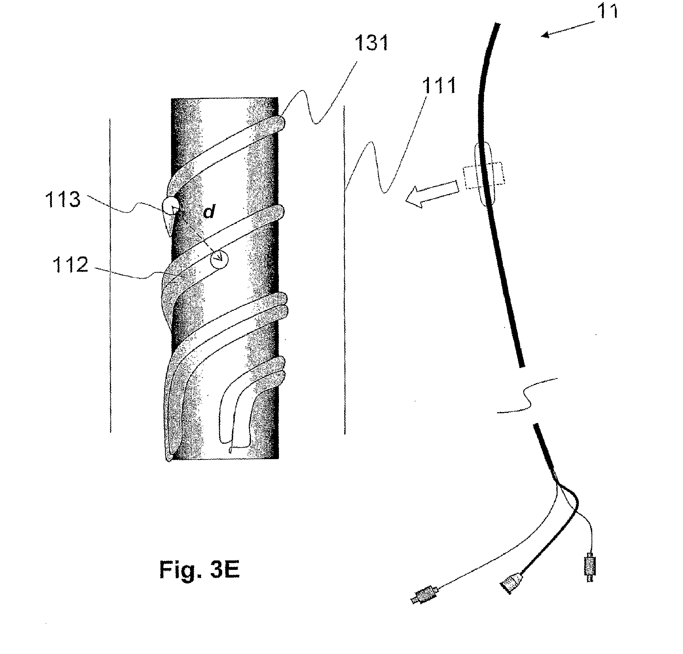

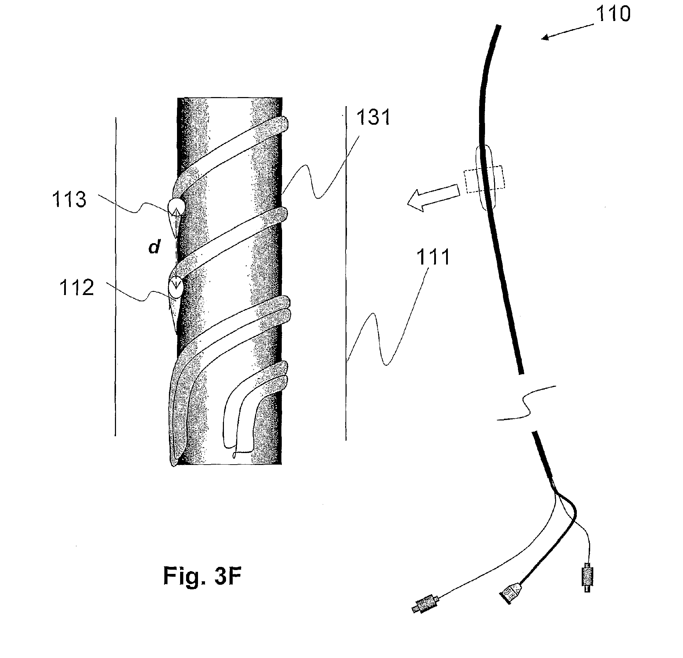

Figs. 3E and 3F are an expanded illustrative view of the catheter 110 of Fig. 3A having four fibers arranged in a helical configuration, wherein the fibers are formed to undergo angular rotation about the core tube 131, wherein the fibers 112, 113 are longitudinally separated and radially separated by a delivery-collection fiber separation ***d***. These two embodiments illustrate fiber separation ***d*** using different fiber arrangements. An optimal ***d*** can be predetermined for collecting an optimal signal from a given depth, or position, of a layer of the target wall . An optimal ***d*** can be calculated using ray tracing algorithms, experimental data and/or using simulation techniques such as Monte Carlo methods. The types of fiber(s), source(s), detector(s), radiation, balloon media, and balloon material, among other factors, will determine how ***d*** and the tissue depth of signals will correlate.

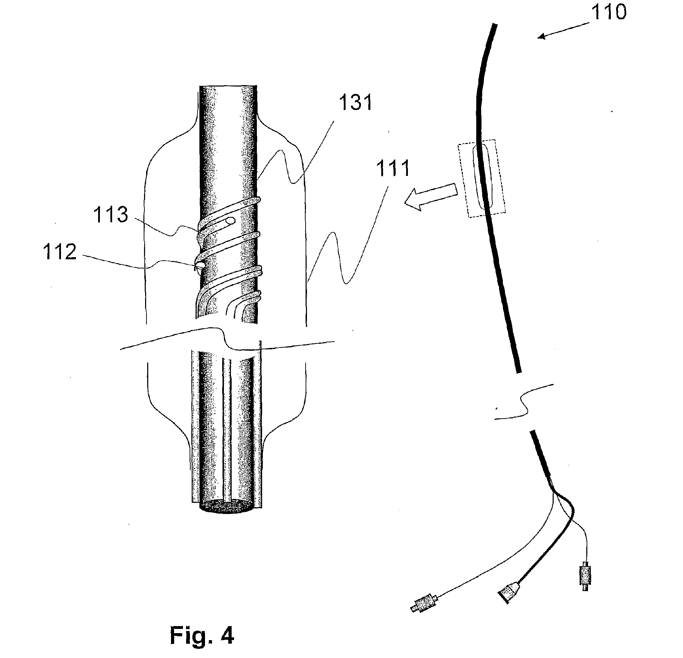

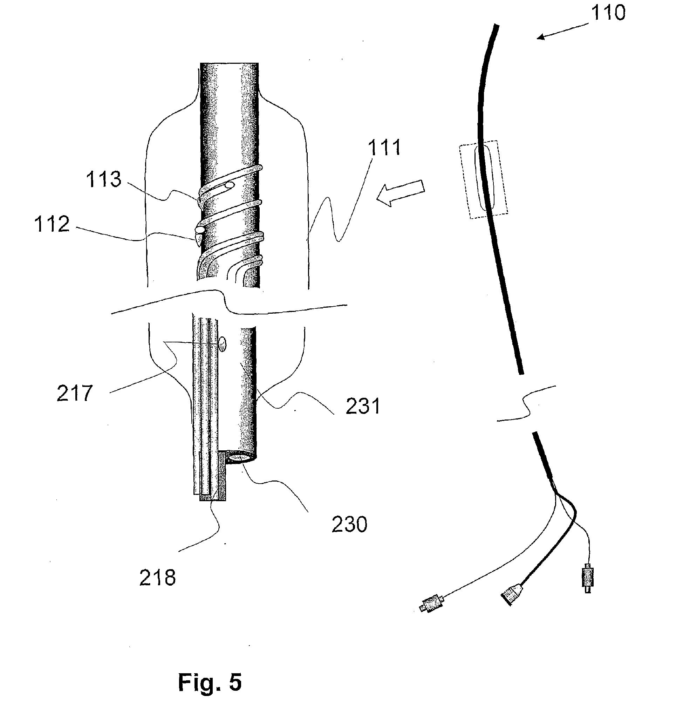

Fig. 4 is an example embodiment of the distal portion of a catheter including four fibers attached to a core tube surrounded by an angioplasty balloon, according to an embodiment of the present invention. In Fig. 4, the balloon 111 is affixed near a distal end of a core tube 131 and coaxially surrounds the distal end of the core tube 131. Fig. 5 is an expanded illustrative view of an example catheter embodiment including four fibers attached to a shortened guidewire sheath 231, which is limited to only the distal portion of the catheter 110 in the region of the balloon 111, according to an embodiment of the present invention. In the embodiment of Fig. 5, the transmission and collection fibers 112, 113 and a transfer tube 218 can be enclosed in a catheter tube (not shown) comprising a flexible sheath or jacket that surrounds the fibers 112, 113 and the transfer tube 218 on the body of the catheter. The balloon 111 coaxially surrounds the distal end of the fibers 112, 113 and a portion of the shortened guidewire sheath 231 and is sealed to the guidewire sheath at first and second ends of the balloon 111. In this manner, the distal end of the guidewire lumen 230 extends beyond the balloon 111 so that a guidewire can be threaded through the guidewire lumen 230, and through a body lumen, to a target region in advance of positioning of the balloon catheter. The balloon catheter can subsequently be inserted and positioned at the target region by following the guidewire, in accordance with conventional angioplasty procedures. The shortened guidewire sheath 231 is beneficial in that the guidewire sheath in the body of the catheter is eliminated, resulting in greater catheter flexibility.

Fig. 6A is an expanded illustrative view of the catheter of Fig. 2A having multiple fibers attached to a movable fiber holder 133, according to an embodiment of the present invention. Fig. 6B is a cross-sectional view of the catheter of Fig. 6A, taken along section lines I-I' of Fig. 6A. In Figs. 6A-6B, the movable fiber holder 133 is positioned about, and is coaxial with, the core tube 131. The delivery and collection fibers 112, 113 are positioned in the grooves 1120 formed in the fiber holder 133 and bonded thereto using a bonding agent. The movable fiber holder 133 and fibers 112, 113 can be advanced and/or retracted back along the longitudinal axis of the core tube and/or rotated about the axis of the core tube 131 by advancing, retracting and rotating the proximal end of the fibers 112 and 113, wherein the distal end of the fibers are attached to the fiber holder 133. The advancement and/or retraction of the fiber holder 133 and the attached fibers permits analysis to be performed over a range of positions in the target regions, without the need for moving the catheter 110, which is usually fixed in place by the expanded balloon 111 during an angioplasty procedure or other therapy. In this manner, rotating the delivery fiber 112 and collection fiber 113 permits data to be received from any area along a 360° section of the lumen wall and longitudinal advancement and retraction permits analysis at a range of positions along the lumen wall.

The fiber holder 133 can be formed of a material similar to those materials commonly used in stents, such as stainless steel, alloy steel and gold. In the embodiment illustrated, the distal ends of the fibers 112, 113 are cleaved and/or polished at an angle, for example, at 45 degrees relative to the longitudinal axis. In this embodiment, the optical radiation is partially or totally reflected at an angle from the tip of the delivery fiber 112, whereby the optical radiation radially exits the delivery fiber 112 through a sidewall, or delivery window, of the delivery fiber 112. This will be described in further detail below, for example, in reference to Fig. 11A..

Figs. 6C-6D illustrate another fiber holding arrangement in accordance with the invention. Fiber holding rings 210 include holes 215 in which fibers 112 and 113 are retained. Holding rings 210 and fibers within them can be affixed in place as shown using a bonding agent 205 or alternatively be allowed to slide or rotate, providing control over longitudinal fiber tip positioning as previously described. The fibers can be affixed to the holding rings 215, for example, using a bonding agent. Together the rings 210 and fibers 112, 113 can be bonded to the core tube 131 using the bonding agent 205, or can be made to slide or rotate relative to the core tube 131, to provide control over fiber tip positioning, as described above.

Fig. 7A is an expanded illustrative view of a catheter embodiment having multiple fibers attached to the removable fiber holder 133, and arranged in a helical configuration, according to an embodiment of the present invention. Fig. 7B is a cross-sectional view of the catheter of Fig. 7A, taken along section lines I-I' of Fig. 7A.

In the example embodiment of Figs. 7A-7B, the fibers 112, 113 are affixed the fiber holder 133, wherein the fiber holder 133 comprises grooves 1120 that are formed along helical paths to guide the fibers 112, 113 into a helically bent position. In one embodiment, the helical paths are formed at angles ranging from 30-180 degrees, or greater, of angular rotation about the fiber holder 133. In this manner, the delivery fibers 112 can direct optical radiation to a target region at any location along the lumen wall along a 360 degree section.

In the illustrative example of Figs. 7A and 7B, the distal ends of the fibers 112, 113 are angularly or radially separated. In an embodiment, the distal ends of the respective fibers are bent at an outward angle, such that the tip of each fiber is directed away from the fiber holder 133, wherein the distal end of the delivery fiber can direct optical radiation in an axial direction to the target region. In another embodiment, the fiber tips are cleaved at an angle, wherein optical radiation radially exits the delivery fibers 112, and radiation is received by the collection fibers 113, through a sidewall, or delivery window, of the respective delivery fibers or collection fibers.

Figs. 7C-7F illustrate a fiber holding arrangement for helically arranged fibers in accordance with embodiments of the present invention. In this embodiment, the delivery and collection fibers 112, 113 are positioned through a first fiber holding ring 210, for example, of the type illustrated above in connection with FIGS. 6C-6D, and retained in place, for example using a bonding agent 205 or a temporary holding mechanism such as a vice (not shown). While fibers 112 and 113 are in a relatively straightened position, a second fiber holding ring 220 is then positioned on the core tube 131 so that the ends of the fibers 112, 113 protrude through holes 225. The second fiber holding ring 230 has angled and oblong shaped holes 235 in which the helically arranged fibers 112 and 113 are to be held in place at an angle a relative to the long axis of the core tube 131.. Referring to Figs. 7E-7G, trace lines 232 represent an approximate side-view outline of holes 235 passing through ring 230 at an angle a. The second holding ring 230 is then simultaneously rotated and moved longitudinally along catheter 131 in accordance with direction arrows 212, such that the ends of fibers 112 and 113 form a helical shape. The positioning of fibers 112 and 113 and angle a may be adapted in order to create a predetermined light delivery and collection pattern about the catheter. A bonding agent 205 can be applied to fix the holding ring 230 in place. Braces (not shown) could alternatively be affixed between the first and second holding rings 210, 220 so as to preserve the helical configuration, while optionally allowing the fiber arrangement to slide and rotate about catheter 131.

Referring to Figs. 7E-7G, an embodiment for a .08 mm wide fiber includes holes 235 having an approximate elliptical shape with a major axis length 242 of .1 mm and a minor axis length of approximately .85 mm. Ring 230 can have an outer diameter 240 of approximately .99 mm and an inner diameter 236 of approximately .62 mm. Adaptations include angles of a between approximately 75 to 85 degrees.. Additional embodiments may adapt the second holding ring 230 to have fiber holes of different dimensions so as to incorporate fibers of different sizes within the same catheter (e.g..08 mm wide delivery fibers with .140 mm wide collection fibers).

Fig. 8A is an illustrative view of an embodiment of a catheter including multiple fibers attached to a movable fiber holder 133, and surrounded by a catheter sheath 138. Fig. 8B is a cross-sectional view of the catheter of Fig. 8A, taken along section lines I-I' of Fig. 8A. In Figs. 8A-8B, fibers 112, 113 are affixed to grooves 1120 formed in a fiber holder 133, and the grooves 1120 are formed, along helical paths about the fiber holder 133. In various embodiments, the helical paths are formed at angles ranging from 30-180 degrees of rotation about the fiber holder 133 and core tube 131. Other angles of helical rotation are equally applicable to the embodiments of the present invention. In one embodiment, the fiber holder 133 and the attached fibers 112, 113 are positioned within a catheter sheath 138. In this manner, the catheter sheath 138 remains fixed, or stationary, while the fiber holder 133 rotates about a longitudinal axis A of the core tube 131 and is advanced and/or retracted inside the balloon 111 along the longitudinal axis A of the core tube 131. Thus, the components of this embodiment that rotate are not rotating directly against a lumen wall of a patient.

In the same manner described above, in this embodiment, the core tube 131, fibers 112, 113, fiber holder 133, balloon 111, and catheter sheath 138 are oriented along the longitudinal axis A, and the fiber holder 133 is translatable along the longitudinal axis A relative to the balloon 111 and the lumen wall. In this manner, spectral measurements of the target region of a lumen can be measured in the region of translation along the length of the longitudinal axis A, and about 360 degrees of rotation about the longitudinal axis A.

Figs. 8C-8E are expanded illustrative views of the catheter of Fig. 8A having multiple fibers attached to the fiber holder in a helical configuration, according to other embodiments of the present invention.

In the embodiment of Fig. 8C, the fibers 112, 113 undergo angular rotation along a helix. Specifically, the fibers 112, 113 are attached to the fiber holder 133, for example, along grooves formed in the fiber holder 130, and undergo about 30 to 180 degrees of angular rotation about the fiber holder 133. Helical paths are formed such that the fibers 112, 113 are longitudinally separated by a predetermined distance d, and are angularly aligned. In this manner, the delivery fibers 112 can deliver optical radiation to two quadrants, or 180 degrees, of the lumen wall, and the collection fibers 113 can receive optical radiation from the two quadrants of the lumen wall. The fiber holder 133 and associated fibers 112, 113 freely rotate about the body of the core tube, in a manner similar to the fiber holder illustrated in Fig. 8A.

The embodiment of Fig. 8D is similar to the embodiments of 8A and 8B above, except that in the present embodiment, a single delivery fiber 112 and a single collection fiber 113 are employed. In this embodiment, the tips of the delivery and collection fibers are longitudinally offset and angularly offset.

The embodiment of Fig. 8E is similar to the embodiment of FIG. 8D above, with the exception that in this example, the tips of the delivery and collection fibers 112, 113 are longitudinally offset, but not angularly offset..

Fig. 9A is a schematic diagram illustrating a cross-section of embodiments of the catheter in the process of performing optical analysis on first and second quadrants of a body lumen, and Fig. 9B is a schematic diagram illustrating performing optical analysis on third and fourth quadrants of a body lumen, in accordance with embodiments of the present invention. In this example, the catheter is placed in position, and the balloon is expanded against the lumen wall 160, substantially blocking the flow of blood through the lumen and from interfering with analysis. In embodiments of the invention, the stretching and expansion of the lumen is therapeutic as in the manner of, for example, an angioplasty. The expansion and stretching can be in preparation (as a pre-dilation procedure) for a subsequent stent delivery in the expanded region so as to facilitate and optimize placement of the stent and ensure its apposition against the lumen wall.

In the sense that the balloon is one that is capable of therapeutically expanding the lumen, it is referred to herein as a "lumen-expanding" balloon. "Therapeutic expansion" of the lumen, as used herein, refers to more than mere anchoring of the balloon in the lumen wall, or hindering or stopping blood flow in the lumen; but further refers to actually expanding, dilating, or stretching the lumen tissue so as to increase the diameter or cross-sectional area of the lumen, as in an angioplasty procedure.

In this embodiment, a source 180 is coupled to an optical switch 181, which, in turn, is coupled to a first delivery fiber 112₁ and a second delivery fiber 112₂. A first detector 171 is coupled to a first collection fiber 113₁, and a second detector 172 is coupled to a second collection fiber 113₂. In this example, lumen wall 160 is partitioned into quadrants: first quadrant I, second quadrant II, third quadrant III, and fourth quadrant IV. An additional optical switch can be used to perform optical analysis on the third and fourth quadrants, without the need for an additional light source, collection fibers, or detectors.

In the example of Fig. 9A, the optical switch 181 is in a first position, wherein the source 180 provides optical radiation to the first delivery fiber 112₁. The first delivery fiber 112₁ directs the optical radiation to a target region on the lumen wall 160. The target region as shown in Fig. 9A is defined by first quadrant I and second quadrant II. The first collection fiber 113₁ receives the reflected optical radiation from first quadrant I of the lumen wall 160, and the second collection fiber 113₂ receives the reflected optical radiation from second quadrant II of the lumen wall 160. The collection fibers 113₁, 113₂ can receive the reflected optical radiation from the lumen wall 160 through a balloon (not shown). The first and second collection fibers 113₁,113₂ transmit the reflected optical radiation received from the first and second quadrants to the first detector 171 and second detector 172, respectively.

In the example of Fig. 9B, the optical switch 181 is in a second position, wherein the source 180 provides optical radiation to the second delivery fiber 112₂. The second delivery fiber 112₂ directs the optical radiation to a target region on the lumen wall 160, the target region being defined in this example by the third quadrant III and fourth quadrant IV. The first collection fiber 113₁ receives the reflected optical radiation from the fourth quadrant IV of the lumen wall 160, and the second collection fiber 113₂ receives the reflected optical radiation from the third quadrant III of the lumen wall 160. The first and second collection fibers 113₁,113₂ transmit the reflected optical radiation received from the third and fourth quadrants to the first detector 171 and second detector 172, respectively.

In this manner, as shown in Figs. 9A-9B, the detectors 171, 172 capture highly accurate signal data from all quadrants, and, as a result, spectral features can be measured about a 360 degree section of the lumen wall using a single light source 180 to generate the optical radiation.

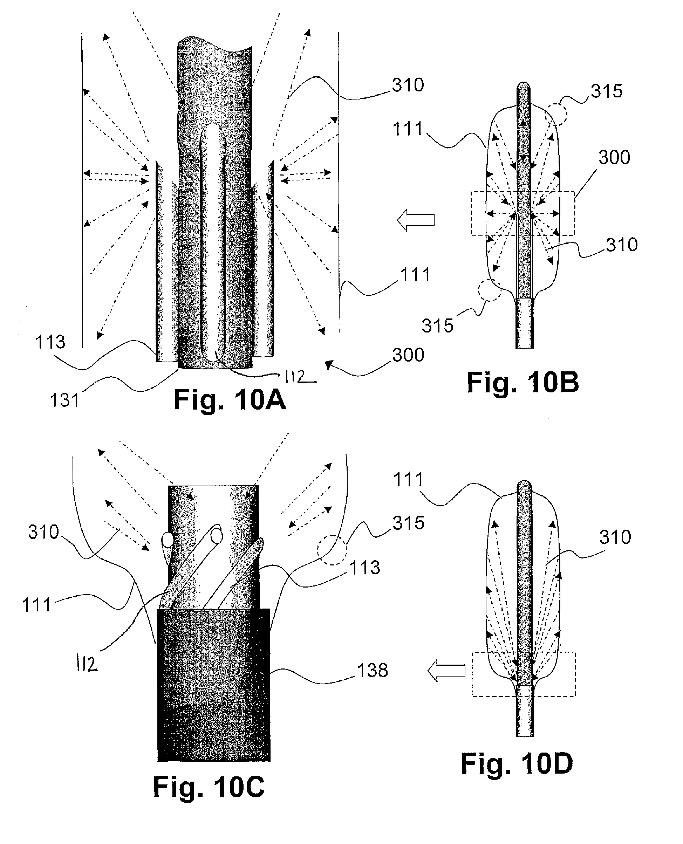

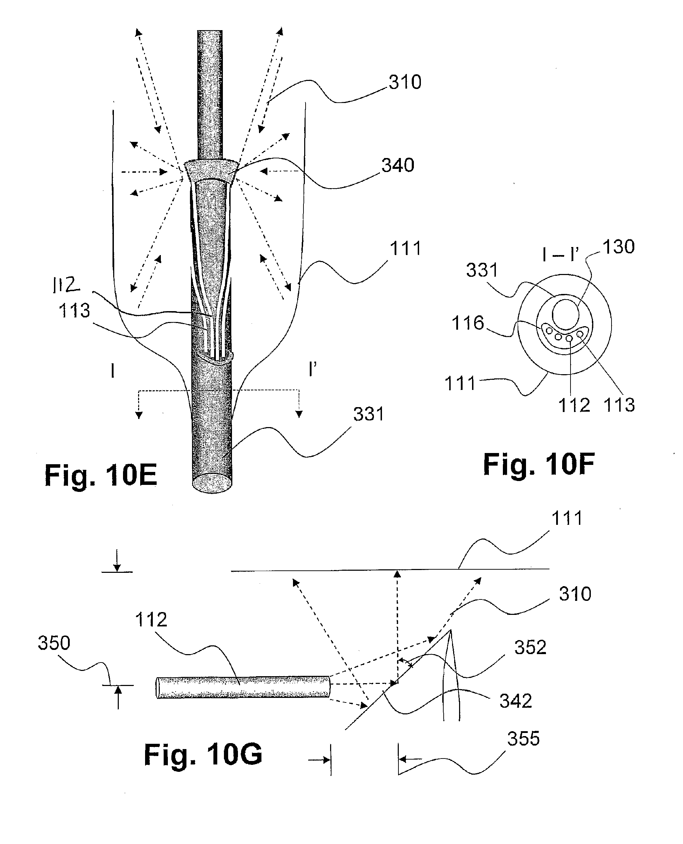

Figs. 10A and 10B show a four fiber catheter arrangement 300, and include sample ray traces of light emission and collection paths from a side-view perspective. Fibers 112, 113 are designed and arranged to project and collect radiation 310 across and through a window of the surface of balloon 111 in contact with the inner surface of a body lumen (not shown). The window extends generally between "elbow" portions 315 of the balloon 111 and about the circumference of balloon 111. The numerical aperture (NA) and the polishing angle of the tips of fiber 113 may be adapted to the shape and size of the (expanded) balloon so that the desired window is achieved. Graded index fibers of high NA, referred to above, are preferred.

Referring to Figs. 10C and 10D, in another embodiment, the receiving and collection ends of fibers 112 of catheter 320 are disposed within the proximate end of balloon 111. This embodiment allows for a stent (not shown) to be crimped about a portion of balloon 111 without underlying fibers 112, and thus reduces the diameter of the crimped stent to allow for easier passage through a lumen. Fibers 112 are arranged to provide light distribution across the surface of balloon 111 as generally shown by ray traces 310.

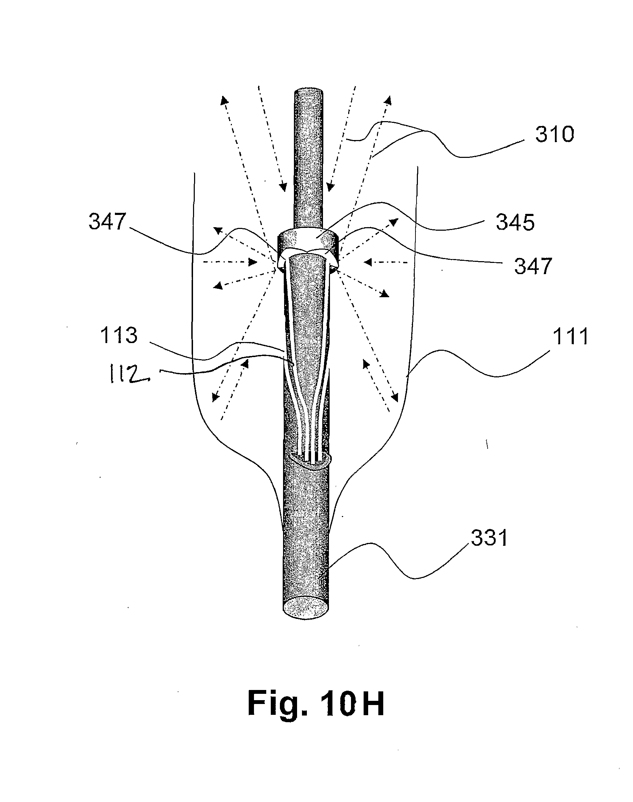

Referring to Figs. 10E-10G, in another embodiment, a single cone-segment reflector element 340 is positioned about a catheter core tube 331 with respect to fibers 112. Reflector 340 may comprise a reflective material such as stainless steel having a polished surface 342. Reflector 340 may alternatively comprise a composite material, such as plastic, that is subsequently coated with a reflective material such as gold. In addition to the numerical aperture of fibers 112, the angle 352 of the reflector 340 can be selected and positions 350 and 355 of fiber 112, relative to the core 342, can be modified so that radiation, as generally depicted by traces 310, is projected across the surface of balloon 111 in a predetermined pattern. Referring further to Fig. 10H, in another embodiment, a multi-faceted reflecting element 345 is provided, including a plurality of reflective facets 347. Facets 347 can be formed in planar or curved shapes similar to or different from each other to further define emission and collection paths about the catheter. A fiber holder or helical arrangement, such as shown in other embodiments of the present detailed description, can be employed to provide control over the positioning of fibers 112 relative to the reflector. Also, in the embodiments of FIGs. 10E and 10H, the delivery and collection fibers 112, 113 are positioned within the fluid transfer lumen 116 of the core tube 331, which terminates upon entering balloon 111. In this example, the guidewire sheath 130 extends adjacent to, and past, lumen 116, and continues to the end of catheter 331.

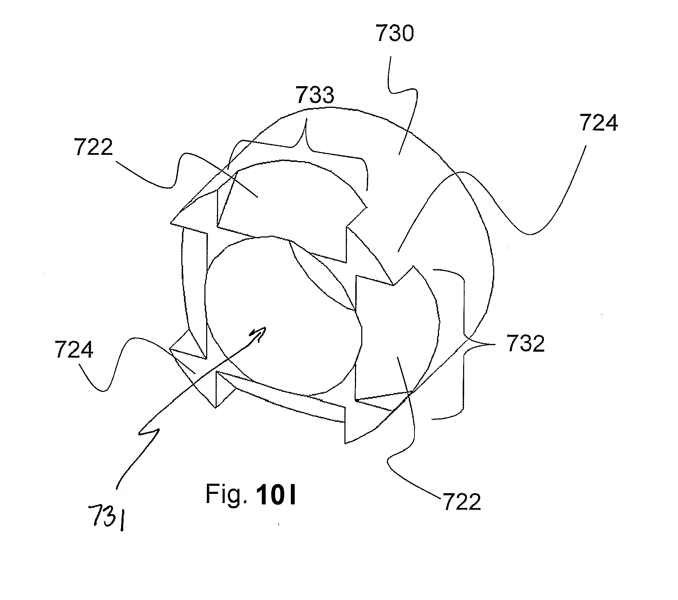

FIG. 10I is a close-up perspective view of an embodiment of the multi-faceted reflector of FIG. 10H. In this embodiment, a reflector body 730 includes a plurality of facets 722. Light-blocking columns 724 are optionally provided between the facets 722 to prevent radiation from inadvertently transmitting between delivery and collection fibers, without first being incident on tissue in the target area. The facets 722 can be sized or shaped to have different lengths or widths, for example, depending on whether the facet is to be associated with a delivery fiber or a collection fiber. For example, a facet 722 designated for a delivery fiber can have a width (i.e. width 733) that is less than a width (i.e. width 732) for a facet 722 to be designated for a collection fiber. A larger sized facet, for example, may be appropriate for association with a collection fiber in order to increase the amount of light collected. The reflector body 730 includes an opening 731 through which the core tube 331 is placed for mounting the reflector body 730 to the core tube 331. Alternatively, the reflector 730 with facets 722 can be formed integral with the core tube 331. In one embodiment, the reflector body 730 can be connected to and/or aligned with a fiber holder, for example a fiber holder 210 of the type described above in connection with FIG. 6C. The fiber holder 210 operates to retain the fibers in alignment with the various corresponding facets of the reflector 730.

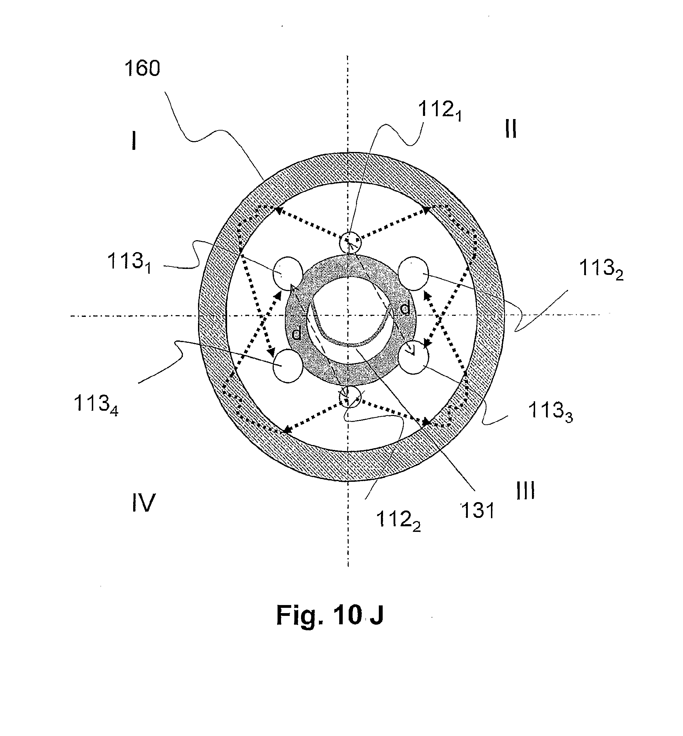

Fig. 10J is a cross-sectional view of a catheter embodiment including six fibers, and performing optical analysis on a body lumen, according to an embodiment of the present invention. In this embodiment, first and second delivery fibers 112₁, 112₂, and collection fibers 113₁ -113₄ are attached to a core tube 131. In alternative embodiments, the first and second delivery fibers 112₁, 112₂ and collection fibers 113₁ -113₄ can be attached to a fiber holder affixed to the core tube 131, as illustrated and described herein. The first delivery fiber 112₁ directs optical radiation to the first quadrant I and second quadrant II of a target region of the lumen wall 160. The fourth collection fiber 113₄ receives the reflected optical radiation from the first quadrant I of the lumen wall, and the third collection fiber 113₃ receives the reflected optical radiation from the second quadrant II. The third and fourth collection fibers 113₃, 113₄ transmit the reflected optical radiation received from the first and second quadrants to respective detectors (not shown).

In addition, the second delivery fiber 112₂ directs optical radiation to the third quadrant III and fourth quadrant IV of a target region of the lumen wall 160. The first collection fiber 113₁ receives the reflected optical radiation from the fourth quadrant IV of the lumen wall, and the second collection fiber 113₂ receives the reflected optical radiation from the third quadrant III. The first and second collection fibers 113₁, 113₂ transmit the reflected optical radiation received from the third and fourth quadrants to detectors (not shown).

The fibers are positioned relative to each other such that the first delivery fiber 112₁ is separated from each of the third and fourth collection fibers 113₃, 113₄ by a distance d, and the second delivery fiber 112₂ is separated from each of the first and second collection fibers 113₁, 113₂ by a distance d. This distance ***d***, also referred to as a delivery-collection fiber separation distance is, in part, determinative of the depth of the path of light into the lumen wall collected by collection fibers 113. Although signals may weaken when the travel path through tissue increases, greater fiber separation provides more information about tissue deeper into the lumen wall 160.

Fig. 11A is an illustrative view of a fiber including an angled tip 122 having a oblique polished end with an applied metal reflective coating 123 and an optical window 124 (known as a "side-fire" arrangement), according to an embodiment of the present invention. Fig. 11B is a cross-sectional view of the instrument of Fig. 11A, taken along section lines I-I' of Fig. 11A. In the example of Figs. 11A and 11B, the fiber cladding 127 in the region of the cleaved tip is removed to provide a delivery or collection window 124 on a sidewall of the fiber through which radially-oriented optical radiation can pass. The coating 123 prevents optical radiation from passing through the tip of the fiber 122 along the longitudinal axis of the fiber 122, and directs the optical radiation to radially, or transversely, exit the side, or body wall of the fiber 122 through the delivery/collection window 124. The fiber 122 is operable as a delivery fiber, in which longitudinally oriented optical radiation from the source is incident on the bevel and reflected radially through the delivery window 124, and the fiber is also operable as a collection fiber which receives reflected optical radiation through the collection window 124 that is reflected by the metal reflective coating 123, and directed longitudinally to a proximal end of the collection fiber.

Fig. 11C is an illustrative view of a beveled fiber tip 122, according to an embodiment of the present invention. In Fig. 11C, a portion of the jacket and cladding 127 of the fiber 122 are removed from the tip of the fiber 122, exposing the fiber core 125. A cylindrical optical window can be affixed to the fiber tip 122 to surround the exposed fiber core 125. In this manner, optical radiation radiates outward from the fiber core through the optical window for a delivery fiber, or radiation is received by the optical window for a collection fiber.

Fig 11D is an illustrative view of an end of a fiber including a transparent capsule, according to an embodiment of the present invention. As illustrated in Fig. 11D, a transparent capsule 134 is affixed to the beveled tip of the optical fiber 122. The capsule 134 is sealed to the fiber tip 122, such that no liquid, gas, or other material that is external to the fiber is in direct contact with the fiber tip 122. Ambient air, or other gas or fluid, can be trapped within the capsule. In this manner, the transparent capsule 134 permits an accurate change in index of refraction to be maintained at the interface of the beveled tip of the fiber during the transmittance of optical radiation to the target region, despite the fiber being immersed in fluid during a procedure. The change in index of refraction ensures suitable internal reflection of the light in the region of the beveled tip. In addition, a portion of the jacket and cladding 127 are removed from the tip of the fiber 122, exposing the fiber core 125. The transparent capsule 134 is affixed about the exposed fiber core 125. In an embodiment, the metal reflective coating is not applied to the fiber tip 122, whereby radiation emitted from the delivery fiber may be axially or radially directed at the target region, or radiation received by the collection fiber may be received axially or radially from the target region. The transparent capsule can be made of an optically suitable material, such as glass, silica, Teflon, or polyimide.

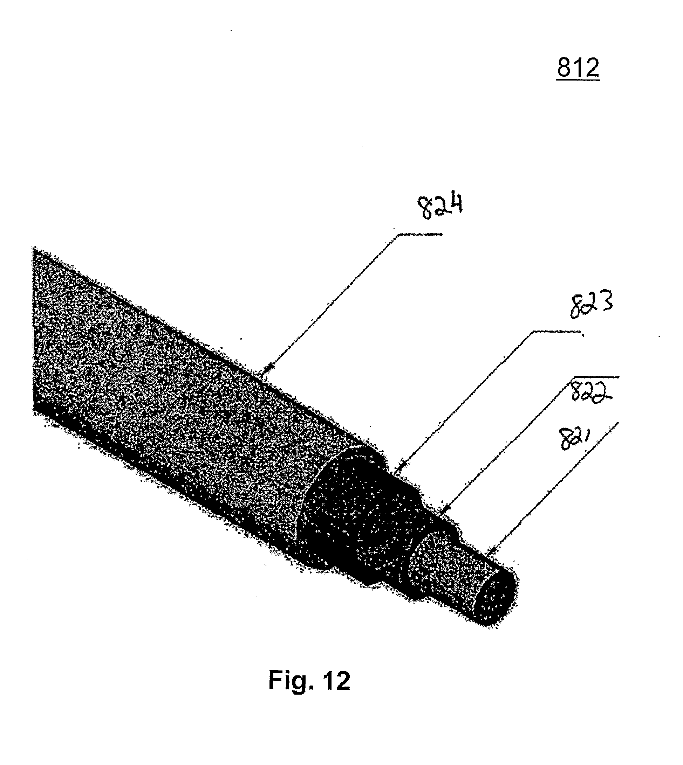

Fig. 12 is a perspective view of a dual-clad optical fiber 812 that can be employed for delivery, collection, or delivery and collection of optical energy, according to another embodiment. In this embodiment, there is no need for a separate delivery fiber and collection fiber, because the dual-clad optical fiber 812 provides the functions of both the delivery fiber and the collection fiber. The dual-clad fiber 812 comprises a primary core 821, a secondary core 822, and a cladding 823 that are enclosed in a protective buffer 824. Optical radiation is transmitted from a proximal end to a distal end of the dual-clad fiber via the primary core 821, wherein the optical radiation exits the distal end. The reflected optical radiation may be collected by the secondary core 822 at the distal end of the fiber 812, wherein the secondary core 822 transmits the reflected and collected optical radiation across the fiber 812 to the proximal end, wherein a detector (not shown) receives the reflected optical radiation. The secondary core 822 is preferably interposed between the primary core 821 and the cladding 823. A dual-clad fiber can also be separated into two individual fibers at its distal end and perform the function of the delivery fiber 112 and collection fiber 113, as described above.

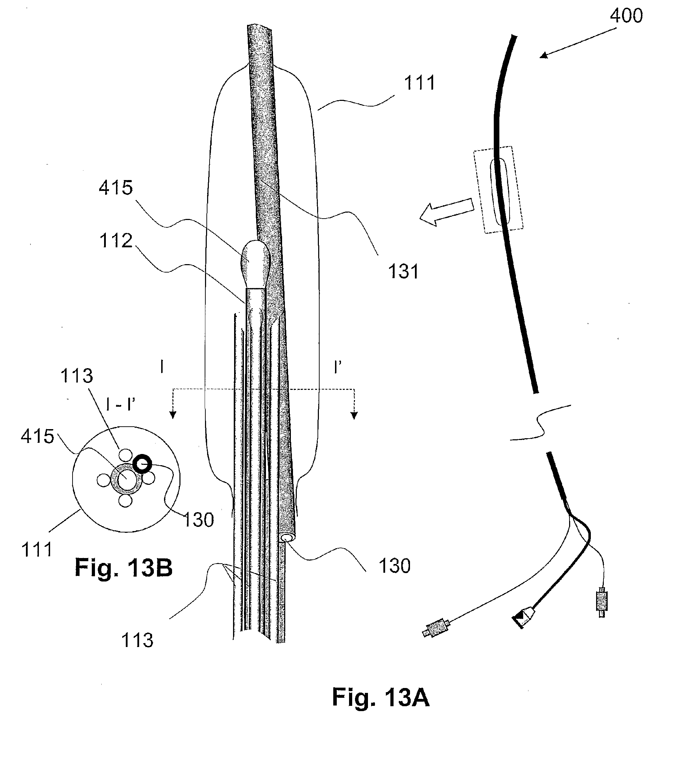

Fig. 13A is an illustrative view of a balloon catheter 400 including a delivery fiber 112 having a diffusing head 415, according to another embodiment of the present invention. Fig. 13B is a cross-sectional view of the instrument of Fig. 13A, taken along section lines I-I' of Fig. 13A, in accordance with the present invention. In the illustrative examples of Figs. 13A and 13B, the diffusing head 415 is mounted to the distal end of a delivery fiber 112. At least one collection fiber 113 is collocated with, and positioned parallel to, the delivery fiber 112. It is preferred that the one or more collection fibers 113 are positioned to receive reflected optical radiation from the lumen wall in any direction, since the optical radiation is transmitted by a source (not shown) from a proximal end to the diffusing head 415 at the distal end of the delivery fiber 112. The optical radiation radiates outward from the diffusing head 415 in all directions toward the lumen wall.

The balloon 111 surrounds the distal ends of the delivery fiber 112 and collection fibers 113, and a portion of the core tube 131. In this manner, the inner surface of the balloon 111 may be illuminated in a 360 degree radius by the optical radiation. Since the diffusing head 415 outputs optical radiation in all directions, and since the core tube 131 is in the path of a portion of the optical radiation, it is preferred that the core tube 131 be composed of absorbing materials that reduce the risk of any optical radiation impinging on the guidewire sheath that may interfere with optical radiation that is intended to be received by the target region at the lumen wall. The "shadow" caused by the guidewire sheath in received radiation can be eliminated as a background by a software program in a data analysis procedure that cancels its effect.

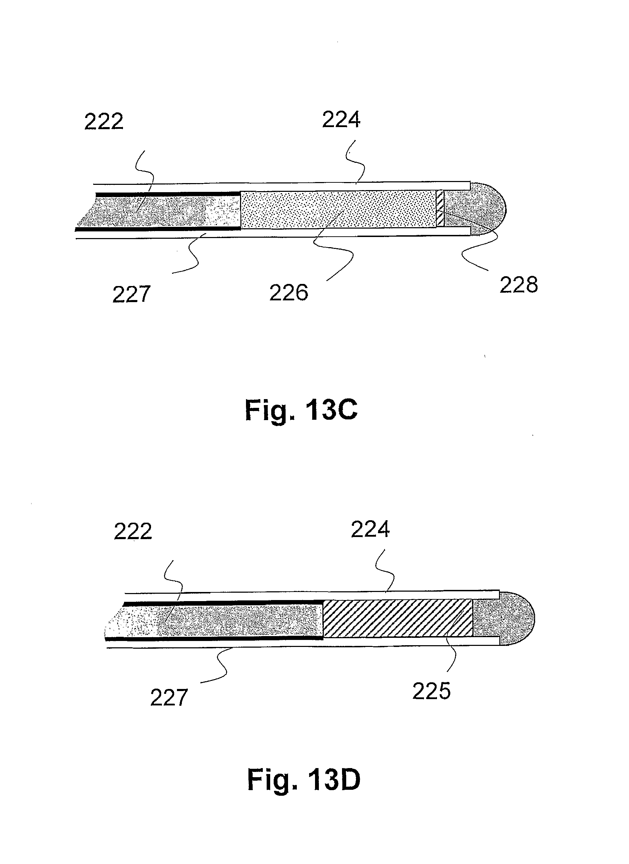

Fig. 13C is an expanded illustrative view of the delivery fiber of Fig. 13A having a reflector 228 and scattering particles 226 according to an embodiment of the present invention. The scattering particles 226 are mounted on the tip of a delivery fiber 112 at the outlet of the core 222 which allows uniform optical radiation along the delivery fiber 112 axis, and reflect optical radiation out of the axis of the delivery fiber 112 through the reflection window 224 to illuminate the lumen wall. The reflector 228 at the end of the scattering particles 226 concentrates the scattering particles 226, thereby interrupting optical radiation in the axial direction. The reflection window 224 can be comprised of high transmission materials such as polymers, silica, and glass.

Fig. 13D is an expanded illustrative view of the delivery fiber of Fig. 13A having a diffuser 225 according to another embodiment of the present invention. The diffuser 225 is positioned at the distal end of the delivery fiber 112 and can diffuse optical radiation from the delivery fiber 112 radially to provide a homogenous illumination of 360° radius area on the lumen wall. The reflection window 224 extends from the fiber sheath 227, and contains the diffuser 225, thereby permitting optical radiation to be transmitted from the diffuser 225.

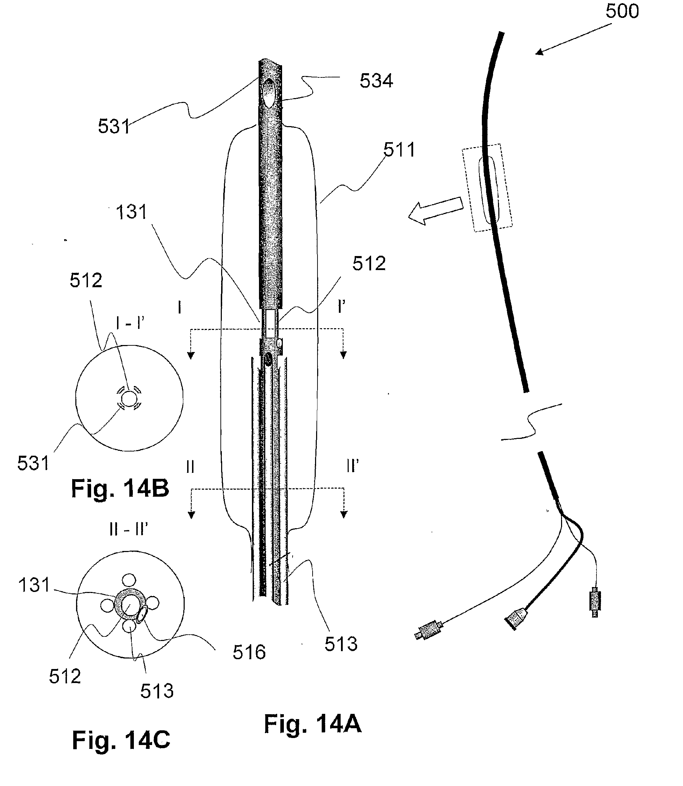

Fig. 14A is an illustrative view of a balloon catheter 500 including a guidewire sheath 531 and a delivery fiber 512 positioned along a longitudinal axis, according to an embodiment of the present invention. Fig. 14B is a cross-sectional view of the instrument of Fig. 14A, taken along section lines I-I' of Fig. 14A. Fig. 14C is a cross-sectional view of the instrument of Fig. 14A, taken along section lines II-II' of Fig. 14A. As shown in Figs. 14A and 14C, a delivery fiber 512 is located inside the core tube 131. In addition, one or more collection fibers 513 are affixed to the outer surface of the core tube 131. In this manner, optical radiation is transmitted along a path of the delivery fiber 512 within the core tube 131. In an embodiment, a diffusing head is positioned at the distal end the delivery fiber 512, for example, a diffusing head of the type illustrated in Figs. 13C-13D. As shown in Fig. 14B, optical radiation can exit the diffusing head at the end of the fiber 512 positioned inside the core tube 131. A second portion of the core tube 131 at the distal end of the balloon 511, includes a guidewire sheath 531 and a guidewire port 534, so that a guidewire (not shown) can be inserted into the guidewire sheath 531.

The first portion of the core tube 131 having the delivery fiber 512 including the diffusing head and the second portion of the core tube 131 having the guidewire sheath 531 and the guidewire port 534 are both positioned along a longitudinal axis. In embodiments illustrated above, the core tube 131 can be composed of plastic or other suitable medium for optical transmission. In the embodiment illustrated in Figs. 14A-14B, the core tube 131 can be composed of material that is not suitable for optical transmissions, wherein a portion of the core tube 131 on the diffusing head at the end of delivery fiber 512 can be removed to form an optical window. In this embodiment, collection fibers 513 are affixed to the first portion of the core tube 131, and the collection fibers 513 each receives reflected optical radiation from the lumen wall. A "shadow" caused by interference with the guidewire, which is located outside of the balloon 511, can be included in the received radiation, which can in turn cause inaccurate results. However, this portion of the radiation can be eliminated by a software program in a data analysis procedure that cancels the effects of the shadow.

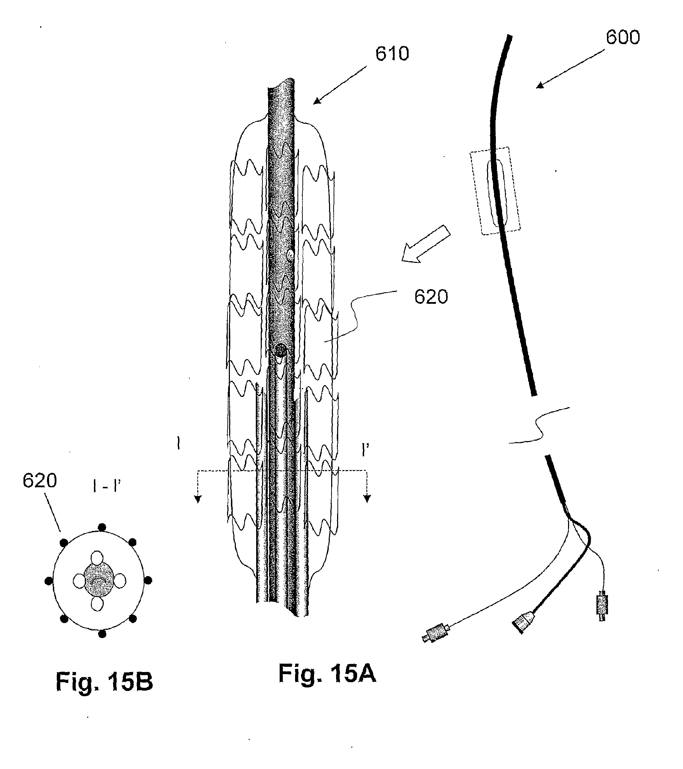

Fig. 15A is an illustrative side view of an instrument 600 used for stent delivery comprising a stent delivery catheter 610, which is similar in form to the balloon catheter described herein, and a stent 620, according to an embodiment of the present invention. Fig. 15B is a cross-sectional view of the instrument 600 of Fig. 15A, taken along section lines I-I' of Fig. 15A, in accordance with the present invention. In a medical procedure, such as an angioplasty procedure including stent insertion, the catheter 610 as illustrated in Fig. 15A is inserted a body lumen to and positioned at a region of the body lumen to be treated. After the stent 620 is inserted in position by expanding the balloon, data relating to spectral features of stented lumen wall is collected through optical fibers. In this manner, the catheter 610 as illustrated in Figs. 15A-15B can also be used as a stent delivery system to perform a stent insertion on the lumen and to perform spectral measurements of the lumen wall, both procedures occurring without the need for removing the catheter 610 from the lumen target region. The instrument 600 can acquire spectra from the lumen wall after the lumen wall is stented. The "shadow" interfered by the stent 620 in received radiation can be eliminated as a background by a software program in data analysis procedure that cancels the effects of the shadow.

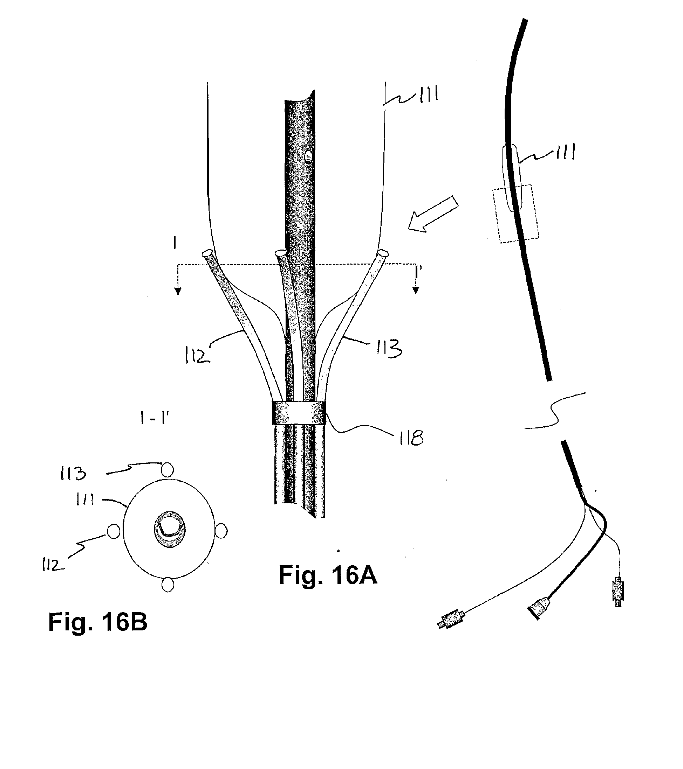

Fig. 16A is an illustrative view of a catheter embodiment including delivery fibers and collection fibers that are adjacent to the outside surface of the balloon 111, according to an embodiment of the present invention. Fig. 16B is a cross-sectional view of the catheter of Fig. 16A, taken along section lines I-I' of Fig. 16A. In an embodiment, the delivery fibers 112 and collection fibers 113 are affixed or molded to the outside surface of the balloon 111. The delivery fibers 112 and collection fibers 113 are pressed against the lumen wall (not shown) by the inflated balloon 111. The balloon 111 coaxially surrounds a portion of the guidewire lumen 130 near a distal end of the guidewire lumen 130. In this manner, optical radiation can be axially or radially directed to the target region. In this embodiment, neither the balloon surface nor the fluid in the inflated balloon is in the path of the transmitted optical radiation. A restriction ring 118 is placed around the fibers, and retains the fibers against the guidewire lumen 130 as the distal ends of the fibers 112, 113 are expanded in an outward direction during inflation of the balloon 111.

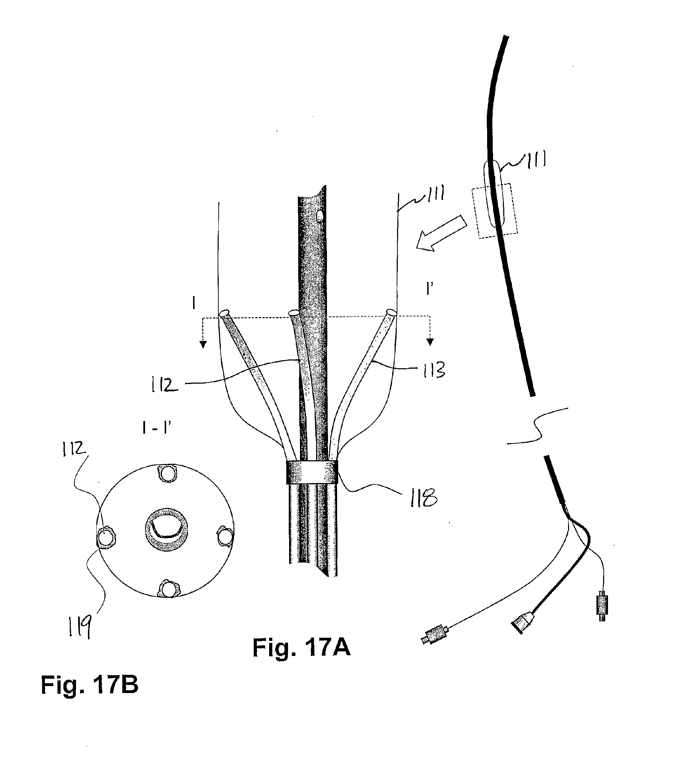

Fig. 17A is a close-up illustrative view of a catheter embodiment including delivery fibers and collection fibers that are affixed or molded to the inside surface of the balloon 111, according to an embodiment of the present invention. Fig. 17B is a cross-sectional view of the catheter of Fig. 17A, taken along section lines I-I' of Fig. 17A. The delivery fibers 112 and collection fibers 113 are affixed or molded to the inside surface of the balloon 111 by an adhesive 119 such as biocompatible ultraviolet glue, tissue adhesive, or epoxy. In this embodiment, optical radiation is axially or radially directed to the target region by passing through the balloon surface; however, the optical radiation is not transmitted, or is minimally transmitted, through the fluid contained in the inflated balloon.

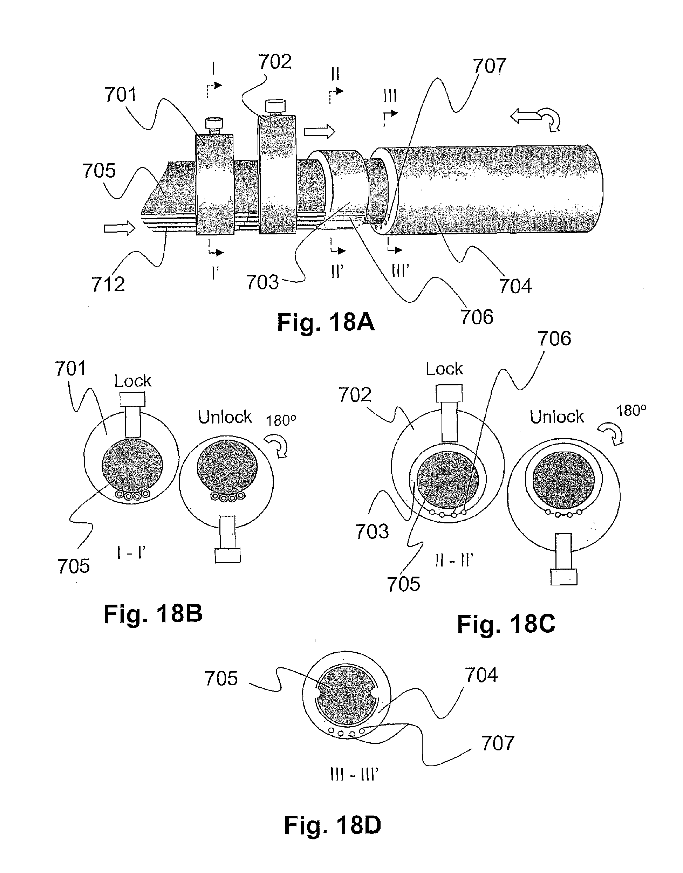

Fig. 18A is an illustrative view of a bending device for helically bending a fiber assembly, in accordance with the present invention. Fig. 18B is a cross-sectional view of the instrument of Fig. 18A, taken along section lines I-I' of Fig. 18A. Fig. 18C is a cross-sectional view of the instrument of Fig. 18A, taken along section lines II-II' of Fig. 18A. Fig. 18D is a cross-sectional view of the instrument of Fig. 18A, taken along section lines III-III' of Fig. 18A.

In Figs. 18A-18D, the bending device helically bends each fiber in the fiber assembly such that a beam of optical radiation can be delivered to a target region of a lumen wall, or collected from a target region of the lumen wall, as illustrated in the various embodiments herein. This device permits both delivery and collection fibers 712 to be helically bent to a specific angle by a heat source (not shown) before the fibers are mounted to a core tube, for example, core tube 131 of Fig. 2A, or a fiber holder, for example, fiber holder 133 of Fig. 6A. The device includes two fiber locking rings 701, 702, a grooved tube 703 with grooves 706, a metal core 705 and a rotating tube 704. The outer jacket on the distal portions of one or more fibers 712 is stripped before the fiber or fibers are mounted to the device for helically bending the fibers. The fibers 712 are inserted through the fiber locking rings 701, 702 and along the metal core 705 and the grooved tube 703. The tips of the fibers are inserted into shallow holes 707 which are located on the facing cross section side of the rotating tube 704 as shown in Fig.18D such that a stripped portion of the fibers (to be helically shaped) extends between grooved tube 703 and rotating tube 704. The fibers are placed on grooves/slots 706 formed along the surface of the grooved tube 703. The fiber locking ring 701 and the fiber locking ring 702 are placed in a locked position as shown in Figs. 18B and 18D respectively. Two fiber locking rings 701, 702 hold the fibers 712 in a fixed position by screws tightened on the fiber locking rings 701, 702. The fiber locking ring 701 holds an unstripped portion of the fibers 701 in a fixed position by clamping the fibers 701 between the metal core 705 and inside portion of ring 701 as shown on Fig. 18B, and the fiber locking ring 702 fixes a stripped portion of the fibers in place by clamping the fibers 712 between the grooved tube 703 and the inside of ring 702 as shown on Fig. 18C. A heating source (not shown) then directs heat of up to about 1600° F on the portion of stripped fiber 712 that extends between the grooved tube 703 (onto which fibers 712 were locked in place by ring 702) and the rotating tube 704.. When the fibers 712 are heated, the rotating tube 704 is rotated and simultaneously advanced towards the grooved tube 703 to a predetermined distance from the grooved tube 703. The heated fibers 712 are bent against the surface of the metal core 705 to form a helical bend having a predetermined angle. The angle of a helical bend of the fibers 712 is dependent on the rotation angle and forward distance of the rotating tube during heating of the fibers. The larger the angle rotated and the longer the distance advanced by the rotating tube 704, the larger the helical bend angle obtained in the treated fibers. The rotated angle of the rotating tube 704 can range, in one example, from 30° to 360°. The distance advanced by the rotating tube can range, in one example, from 2 microns to 2 millimeters, depending on the size of the treated fibers. A helical flange on the surface of the metal core 705 against the inside lumen of the rotating tube 704 can assist in controlling the rotating angle and distance advanced by the rotating tube 704. After the fibers are helically bent, the screws on the fiber locking ring 701 and the fiber locking ring 702 are loosened. Both the fiber locking ring 701 and the fiber locking ring 702 are rotated 180°. The bent fibers 712 are removed from the both locking ring windows. The resulting bent fibers 712 can then be mounted to, for example, the core tube 131 or the fiber holder 133 of previously described embodiments.

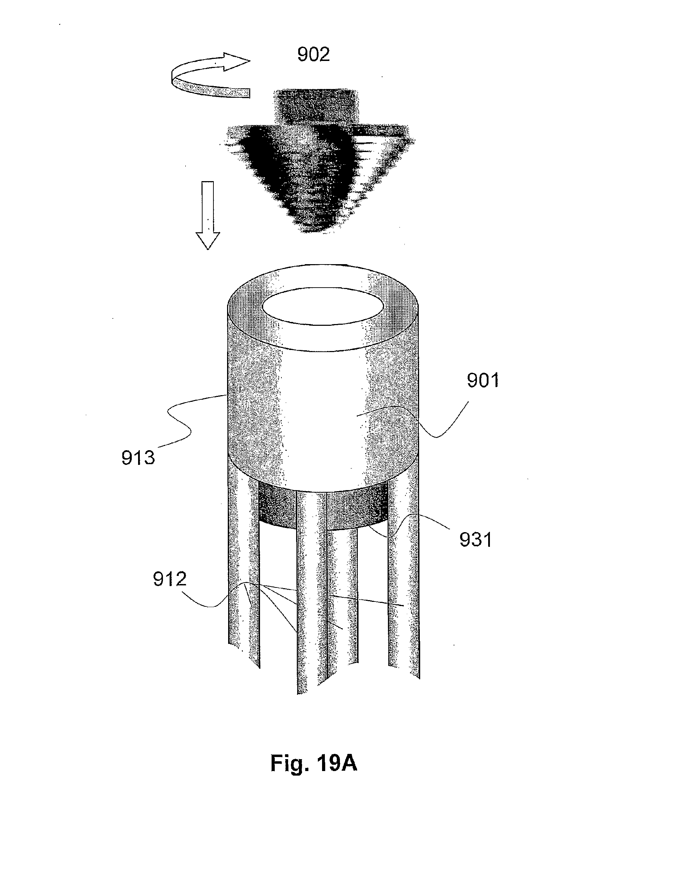

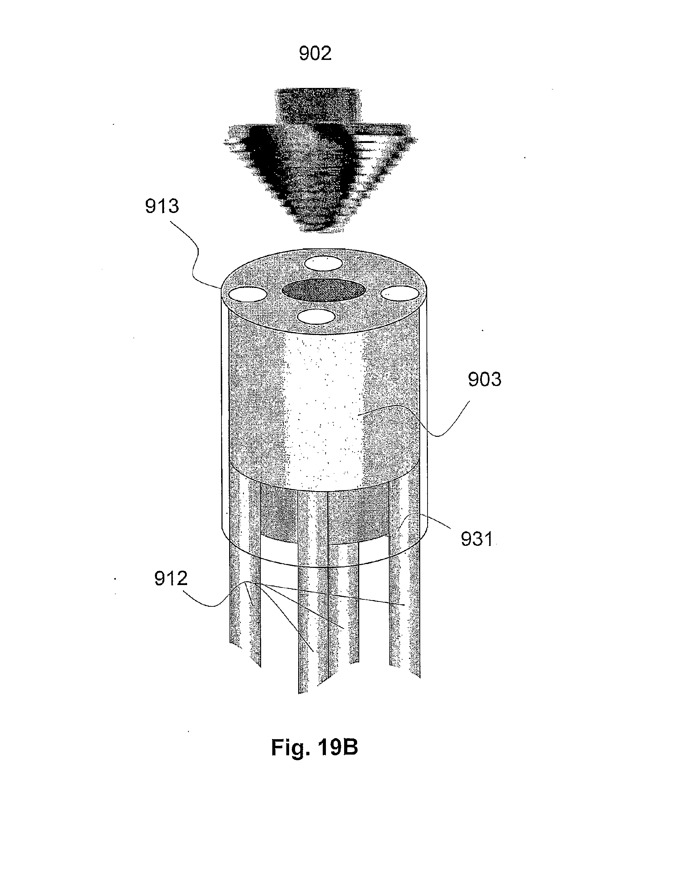

Figs. 19A-19D are illustrative views of sequential steps of an instrument manufacturing process, in accordance with an embodiment of the present invention. This method provides for both delivery and collection fibers 912 to be obliquely polished to the same angle using a rotating shaper 902 in the manufacturing of a catheter. As shown in Fig. 19A, a plurality of fibers 912 are positioned to be parallel to each other around a fiber holder 931. Fiber holder 931 can be of the type, for example, as those described in reference to Figs. 6A-6D among others. Each fiber 912 is affixed to the fiber holder 931 using glue or epoxy (not shown). The fiber tips 913 extend beyond the fiber holder 931; however, the blunt fiber tips 913 can be completely covered by the glue or epoxy for protection, as well as to affix the fiber tips 913 to the holder 931. A metal ring 903 may be optionally positioned over the distal end of the fiber tip 913 and glue or epoxy. As shown in Fig. 19B, the metal ring 903 can temporally protect the fiber tips 913 when fiber tips 913 are obliquely polished by the shaper 902.

As shown in Fig. 19B, the shaper 902 rotates at a suitable rotating speed, ranging from 1000-100,000 rpm. The shaper 902 is slowly applied to the fiber tips 913 wrapped by glue or epoxy 901 and protected by the protection 903, to obliquely polish the fiber tips 913 by the shaper 902.

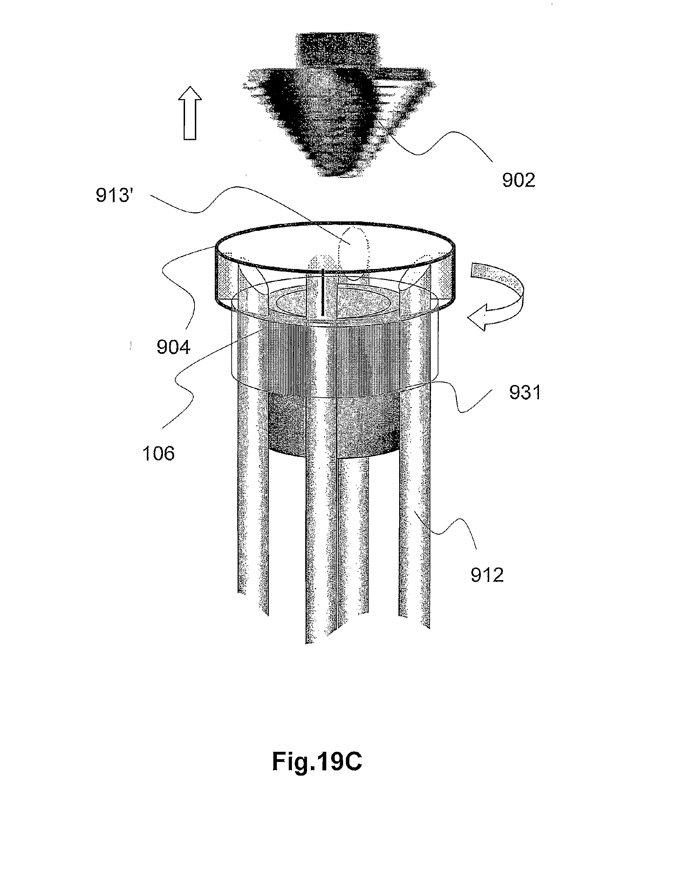

As shown in Fig. 19C, after the fiber tips 913' are obliquely polished to an angle, e.g. 45 degrees, the protection ring 903, which protects the fiber tips during subsequent polishing steps, is removed. A window cutter 904 is positioned around the fiber tips 913' to strip the fiber cladding and jacket from the tips as well as glue or epoxy 901 coating the tips to form an optical window for each fiber tip 913'. The oblique sides of the fiber tips 913' are then coated with metal material, such as gold, nickel and aluminum. The tip-polished fibers 912 affixed to the fiber holder 931 with remaining glue or epoxy 106 are then mounted to a core tube (not shown) .

An angle and polish shaper 902 is applied to the angled fiber tips 913', whereby the tips 913' are further shaped and polished to achieve an accurate, optimum fiber tip angle having desired integrated optical properties, such as high-reflectance properties.

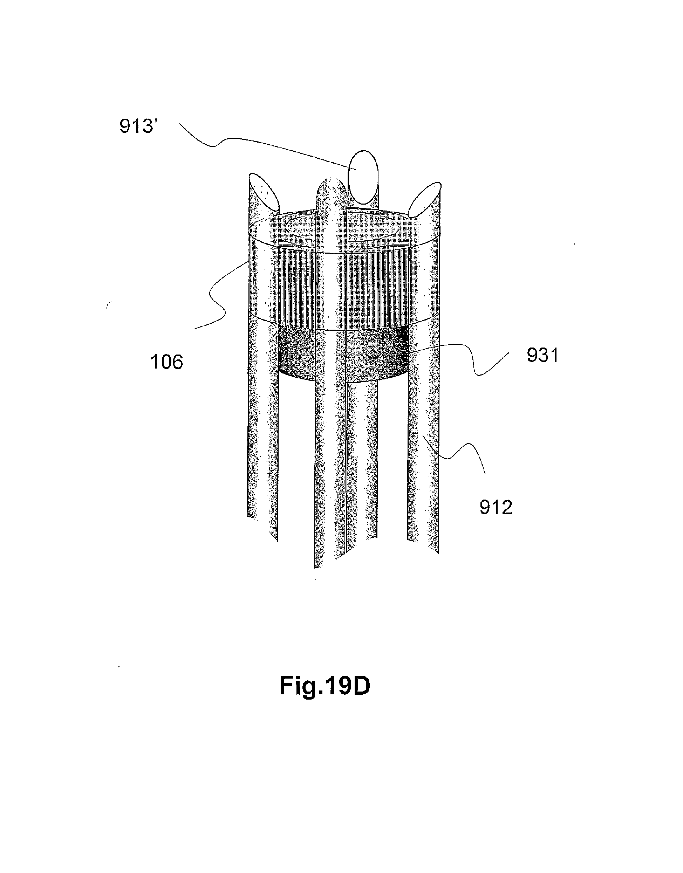

As shown in Fig. 19D, a catheter 910 comprising optical fibers 912 having angled fiber tips 913' is formed. A balloon, source, detector, and spectrometer may subsequently be attached to the catheter 910 in a conventional sequence to complete the balloon catheter manufacturing process.

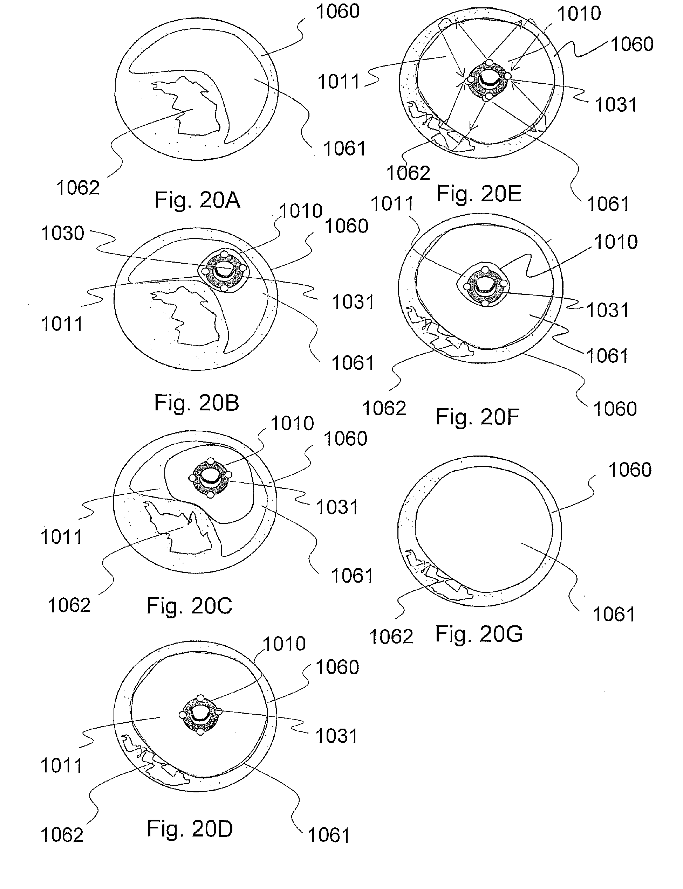

Figs. 20A -20G are cross-sectional views illustrating the sequential steps of performing a balloon angioplasty procedure, in accordance with an embodiment of the present invention. Fig. 20A is a cross-sectional view of a constricted body lumen 1061 having a lumen wall 1060. The lumen 1061 may be constricted due to a blockage, for example a blockage 1062 caused by an accumulation of lipid content.

As shown in Fig. 20B, a balloon catheter 1010, for example of the type described herein, is inserted into the constricted lumen 1061 in accordance with conventional procedures. In one embodiment, the balloon catheter 1010 comprises a core tube 1031 including a guidewire lumen 1030, a balloon 1011, and at least one delivery/collection fiber. During a treatment procedure, the physician first inserts a guidewire into the constricted lumen 1061 via a puncture point located at the groin or wrist. Next, the physician places the balloon catheter 1010 on the guide wire. The balloon catheter 1010 comprises a balloon 1011 that, upon entry to the constricted lumen 1061, is in a deflated state.

As shown in Fig. 20C, the positioned balloon 1011 is partially inflated by delivering fluid through a port in the core tube 1031 into the balloon 1011. The catheter 1010 enables the collection of data of the spectral features of the lumen wall 1060 by delivering optical radiation from the delivery fiber to the lumen wall, and collecting optical radiation that is emitted from the lumen wall and received by the collection fiber. The collection of data of the spectral features of the lumen wall are used to determine the position of the balloon catheter 1010 with respect to a target region. Since the lumen wall information is obtained via spectral analysis in real-time, the physician can rely on this information to determine where to place the catheter 1010 with regard to an area of interest, for example, a diseased area of the lumen, and, accordingly, to perform the necessary procedure (e.g. balloon angioplasty and/or stent insertion).

This feature is advantageous over conventional catheters, for example, catheters relying on fluoroscopy, since fluoroscopy merely enables a user to guide the conventional catheter to the diseased area. However, fluoroscopy can only provide information on a diseased area of a lumen in two-dimensions (e.g. blood vessel stenosis in a two-dimensional cross-section), and therefore an incomplete analysis is provided. This is particularly important in certain applications, wherein some disease regions may require a treatment, but conventional methods involving fluoroscopy cannot identify vulnerable plaque in a non- or minor stenosis area. Since the present invention can also identify weaknesses along the lumen wall prior to deploying an angioplasty balloon at a target region of the lumen wall, the present invention can reduce the risk of a rupture occurring at or near the blockage 1062 during or after the angioplasty procedure.

In another embodiment, the catheter 1010 collects data on the spectral features of the balloon 1011. This data can be used to determine the distance of the balloon surface from the guidewire lumen 1030 during inflation or deflation of the balloon 1011 or to measure the volume of expansion of the balloon 1011 during inflation.

As shown in Fig. 20D, after the catheter 1010 is placed in a region of an identified disease position, the balloon 1011 is fully inflated, thereby dilating the lumen 1061 at the target region for a treatment of balloon angioplasty and/or stent insertion (stent not shown). The pressure of the inflated balloon 1011 against the lumen wall 1060 is sufficient to obstruct blood flow, and to displace any blood in the path between the outer surface of the balloon 1011 and the lumen wall 1060 to be measured. The balloon can be pressurized, for example, to a pressure in the range of about 8 - 12 atmospheres, and, in this state, can be of a substantially cylindrical form within a lumen.

After or during a therapy such as a balloon angioplasty treatment, another collection of spectral features can be performed. As shown in Fig. 20E, optical radiation is transmitted from a distal end of the delivery fiber and transmitted through the balloon 1011 to the balloon surface that abuts the lumen wall 1060. The optical radiation passes through the balloon surface and impinges the target region of the lumen wall 1060 and can interact with the tissue/fluids therein in the manner of, for example, fluorescence, luminescence, and/or diffuse reflectance. Collection fibers can receive the emitted optical radiation from the lumen wall 1060 that passes thorough the balloon 1011. The emitted optical radiation is received by one or more detectors, which generate signals from the received optical radiation. The signals can be processed by a spectroscopic analysis system, wherein the processed signals are stored and presented to the user via a computer or display. Since the balloon 1011 is in direct contact with the lumen wall, such that little or no blood is between the balloon and the lumen wall, high-quality spectral data can be obtained. This additional spectral data allows the physician to receive in real-time the treatment results, as well as current physiological and pathological changes on the treatment. The physician can rapidly make a decision for subsequent therapy, e.g. a stent insertion and/or a drug local injection therapy after a sample balloon angioplasty for second treatment. The spectral data can also indicate the preferred stent to be selected for treatment, of any required future treatment, etc. by analyzing pathology results on the lumen wall. The spectral data can also be stored for future analysis or comparison to current treatment(s).

As shown in Fig. 20F, after treatment and spectral data collection, the balloon 1011 is deflated, whereby the fluid is removed from the balloon 1011 through a flush port located in the core tube 1031.

As shown in Fig. 20G, the balloon catheter 1010 is removed from the lumen 1061. The lumen 1061 has an opening that is significantly increased as a result of the balloon angioplasty or stent insertion (not shown).

Embodiments of the invention can thus integrate the gathering of critical information about vessel walls with many therapies for blocked/diseased vessels, including lumen-expansion therapy and stenting. For example, because an area that is targeted for a stent procedure is often obstructed, it is commonly preferable to have a pre-dilation step (a step that occurs in about seventy percent of all stenting procedures) in which an angioplasty balloon is deployed without a stent and expanded within the vessel to initially unblock the targeted area. This pre-dilation step facilitates and optimizes placement of the stent and helps ensure apposition against the vessel. The use of embodiments of the present invention with this pre-dilation step will greatly enhance the amount of information gathered prior to insertion of a stent. This information can include improved estimates of whether or not a stent is the preferred course of treatment, the position, type and size of the stent, if any, to be deployed, and the preferred type of coatings on the stent and/or drugs to be eluted from the stent.

It will be understood by those with knowledge in related fields that uses of alternate or varied forms or materials and modifications to the methods disclosed are apparent. This disclosure is intended to cover these and other variations, uses, or other departures from the specific embodiments as come within the art to which the invention pertains.

### Further Embodiments:

The present invention also provides for a catheter for placement within a body lumen, the catheter comprising: a flexible conduit that is elongated along a longitudinal axis, the flexible conduit having a proximal end and a distal end; at least one delivery waveguide and at least one collection waveguide positioned along the flexible conduit, the at least one delivery waveguide and the at least one collection waveguide constructed and arranged to transmit radiation at a wavelength in a range of about 250 to 2500 nanometers; and a lumen-expanding inflatable balloon disposed about a portion of the conduit, a transmission output of the at least one delivery waveguide and a transmission input of the at least one collection waveguide located within the balloon.

The lumen-expanding balloon may comprise an angioplasty balloon. The transmission output of the at least one delivery waveguide and the transmission input of the at least one collection waveguide may be contiguously retained to the flexible conduit.

The catheter may further comprising a fiber holder disposed about the conduit that contiguously retains the transmission output of the at least one delivery waveguide and the transmission input of the at least one collection waveguide to the flexible conduit. The fiber holder may comprise at least one holder body having a plurality of holes that are substantially aligned with the longitudinal axis of the conduit when mounted thereto, the at least one delivery waveguide and the at least one collection waveguide being secured to the fiber holder at the holes. The fiber holder may comprise at least one holder body having a plurality of grooves on a surface thereof, the at least one delivery waveguide and the at least one collection waveguide being secured to the fiber holder at the grooves.

The plurality of grooves may be arranged in a helix. The plurality of grooves may be substantially aligned with the longitudinal axis of the conduit when the fiber holder is mounted thereto. The fiber holder may be longitudinally translatable relative to the longitudinal axis of the flexible conduit so that the transmission output of the at least one delivery waveguide and the transmission input of the at least one collection waveguide are translatable between a first longitudinal position and a second longitudinal position of the conduit. The fiber holder may be rotatable about the longitudinal axis of the flexible conduit so that the transmission output of the at least one delivery waveguide and the transmission input of the at least one collection waveguide can be rotated about the conduit.

The transmission output of the at least one delivery waveguide and the transmission input of the at least one collection waveguide may be spaced apart at a predetermined distance in order to facilitate collection of radiation emitted from tissue of a predetermined depth from the lumen-expanding inflatable balloon through the transmission input. The at least one delivery waveguide may comprise at least one delivery fiber optic and wherein the at least one collection waveguide comprises at least one collection fiber optic. The at least one delivery fiber optic may have a tapered end that operates as a reflection surface for changing a direction of a path of radiation transmitted along a longitudinal axis of the delivery fiber optic so that the radiation is emitted in a direction that is transverse to the longitudinal axis of the fiber. The at least one collection fiber optic may have a tapered end that operates as a reflection surface for changing a direction of a path of radiation transmitted into the transmission input of the collection fiber optic so that the radiation is transmitted along a longitudinal axis of the collection fiber optic.

The catheter may further comprise an optical element disposed about the flexible conduit, the optical element including an array of multiple facets that lie at an acute angle relative to the longitudinal axis of the flexible conduit for changing a direction of radiation transmitted along a longitudinal axis of the at least one delivery waveguide so that the radiation is emitted in a direction that is transverse to the longitudinal axis of the at least one delivery waveguide. The catheter may further comprise an optical element disposed about the flexible conduit, the optical element including an array of multiple facets that lie at an acute angle relative to the longitudinal axis of the flexible conduit for changing a direction of radiation transmitted into the transmission input of the at least one collection waveguides so that the radiation is transmitted along longitudinal axes of the collection waveguides.

The distal ends of the at least one collection waveguides in the region of the transmission input may lie along a helical path about the longitudinal axis of the conduit. The distal ends of the at least one delivery waveguides in the region of the transmission output may lie along a helical path about the longitudinal axis of the conduit.

The transmission output of the at least one delivery waveguide and the transmission input of the at least one collection waveguide may be spaced apart at a predetermined distance in a longitudinal direction along the longitudinal axis of the conduit. The balloon comprises a polymer material that is substantially transparent to radiation at the wavelength in the range of about 250 to 2500 nanometers. The polymer material is selected from the group of materials consisting of nylon and polyethylene.

The at least one delivery waveguide may comprise a plurality of delivery waveguides and wherein the at least one collection waveguide comprises a plurality of collection waveguides. The at least one delivery waveguide may comprise two, three or four delivery waveguides and wherein the at least one collection waveguide may comprise two, three or four collection waveguides.

The plurality of transmission outputs of the plurality of delivery waveguides may be arranged to illuminate an interior wall of a lumen about a 360 degree portion thereof through the balloon, when the balloon is inflated within the lumen, and wherein the plurality of transmission inputs of the plurality of collection waveguides may be arranged to receive radiation from the interior wall of the lumen about the illuminated 360 degree portion thereof through the balloon.

The at least one delivery waveguide may comprise first and second delivery waveguides and wherein the at least one collection waveguide may comprise first and second collection waveguides, and wherein the transmission outputs of the first and second delivery waveguides may be positioned circumferentially opposite each other relative to the flexible conduit and wherein the transmission inputs of the first and second collection waveguides may be positioned circumferentially opposite each other relative to the flexible conduit, so that four quadrants of a 360 degree portion of an interior wall of the lumen can be illuminated by the radiation through the balloon and so that reflected radiation can be received from the four quadrants of the interior wall through the balloon.

The transmission output of the at least one delivery waveguide may comprise an uncladded fiber core sealed within a covering that is substantially transparent to radiation at the wavelength in the range of about 250 to 2500 nanometers. The substantially transparent covering may comprise a cylindrical capsule containing a material having an index of refraction so as to provide an interface between the uncladded fiber core and the material in the capsule to direct incident radiation in a predetermined direction. The transmission output of the at least one delivery waveguide may comprise scattering particles and a reflective terminating member so as to direct radiation in a direction that is transverse to a longitudinal axis of the at least one delivery waveguide.

The balloon may be sealed to the flexible conduit at a first longitudinal position and the second longitudinal position of the flexible conduit. The balloon may be coupled to the conduit at a first longitudinal position of the conduit at a first portion of the balloon and wherein the balloon is coupled to the conduit at a second longitudinal position of the conduit at a second portion of the balloon, and wherein the transmission output of the at least one delivery waveguide and the transmission input of the at least one collection waveguide may be located within the balloon between the first and second longitudinal positions of the conduit.

The catheter may further comprise a guidewire sheath coupled to the conduit at the distal end of the conduit, wherein the balloon is coupled to the guidewire sheath and conduit at a first portion of the balloon and wherein the balloon is coupled to the guidewire sheath at a second portion of the balloon. The flexible conduit may comprise a core tube including a guidewire lumen.

The at least one collection waveguide and the at least one delivery waveguide may be positioned within a fluid transfer lumen of the core tube along a majority of its length. The at least one collection waveguide and the at least one delivery waveguide are positioned within a catheter sheath surrounding the core tube along a majority of its length.

At least one of the at least one delivery waveguide and the at least one collection waveguide may comprise graded-index optical fiber. At least one of the at least one delivery waveguide and the at least one collection waveguide has a numerical aperture between approximately .22 and.4. The at least one delivery waveguide may comprise a fiber having a fiber core diameter of between about 9 and 100 microns. The at least one collection waveguide may comprise a fiber having a fiber core diameter of between about 50 and 200 microns.

The at least one delivery waveguide may comprise a fiber having a fiber core diameter of about 50 microns and wherein the at least one collection waveguide may comprise a fiber having a fiber core diameter of about 100 microns. A maximum outer diameter of the catheter including the flexible conduit, the at least one delivery waveguide, the at least one collection waveguide and the balloon may be less than about 1.5 millimeters when the balloon is uninflated.

The present invention also provides for a system for probing and treating a body lumen comprising: a flexible conduit that is elongated along a longitudinal axis suitable for insertion into a body lumen, the conduit having a proximal end and a distal end; at least one delivery waveguide and at least one collection waveguide integrated with the flexible conduit; at least one radiation source connected to a transmission input of the at least one delivery waveguide, the radiation source constructed and arranged to provide radiation at a wavelength in a range of about 250 to 2500 nanometers; at least one optical detector connected to a transmission output of the at least one collection waveguide; and a lumen-expanding inflatable balloon disposed about a portion of the conduit, a transmission output of the at least one delivery waveguide and a transmission input of the at least one collection waveguide located within the balloon.

The transmission output of the at least one collection waveguide may be connected to a spectrometer, the spectrometer constructed and arranged to scan radiation and perform spectroscopy at the wavelength in the range of about 250 nm to 2500 nm. The spectrometer may be configured to perform spectroscopy selected from the group of spectroscopy methods consisting of fluorescence, light scatter, optical coherence reflectometry, optical coherence tomography, speckle correlometry, Raman, and diffuse reflectance spectroscopy. The spectrometer may be constructed and arranged to scan radiation and perform spectroscopy at a wavelength within the range of about 750 nm to 2500 nm.

The spectrometer may be constructed and arranged to scan radiation and perform spectroscopy using one or more ranges of wavelengths. A scan using the one or more ranges of wavelengths may include a scan using one or more discrete wavelengths. The system may further comprise a controller that is programmed to automate control of activation and deactivation of the at least one radiation sources and the at least one optical detectors, to further control analysis of data collected by the system.

The system may be constructed and arranged for use in a medical care facility including a hospital or outpatient unit. The controller may be programmed to operate a human- interactive interface that provides an operator with feedback about data and analysis of the spectroscopy, the interface providing information for real-time diagnosis. The controller may be programmed to identify one or more characteristics of targeted tissue including at least one of: presence of chemical components, tissue morphological structures, water content, blood content, temperature, pH, and color.

The controller may be further programmed to discriminate between tissue characteristics and non-relevant artifacts including elements of the catheter and other elements artificially introduced into the body lumen. The artificially introduced elements may include at least one of stents and the coatings of stents. The system may further comprise a switch coupled between the at least one radiation source and the at least one delivery waveguide that selects between multiple radiation sources for application of radiation to the at least one delivery waveguide.

The system may further comprise a switch coupled between the at least one radiation source and the at least one delivery waveguide that selectively applies the at least one radiation source to the at least one delivery waveguide. The system may further comprise a therapy delivery subsystem. The therapy delivery subsystem may further comprise a tube associated with the flexible conduit through which at least one of treatment drugs and agents can be delivered. The one or more radiation sources may be configured to produce an output power of the radiation of less than about 20 milliwatts at locations outside the balloon when inflated.

The invention also provides for a catheter for placement within a body lumen, the catheter comprising: a flexible conduit that is elongated along a longitudinal axis, the flexible conduit having a proximal end and a distal end; at least one delivery waveguide and at least one collection waveguide positioned along the flexible conduit; and a lumen-expanding inflatable balloon disposed about a portion of the conduit, a transmission output of the at least one delivery waveguide and a transmission input of the at least one collection waveguide located within the balloon, wherein the maximum outer diameter of the catheter, including the flexible conduit, the at least one delivery waveguide, the at least one collection waveguide and the balloon is less than about 1.5 millimeters when the balloon is uninflated.

The at least one delivery waveguide and the at least one collection waveguide may be constructed and arranged to transmit radiation at a wavelength in a range of about 250 to 2500 nanometers. At least one of the at least one delivery waveguide and the at least one collection waveguide may comprise graded-index optical fiber. At least one of the at least one delivery waveguide and the at least one collection waveguide may have a numerical aperture between approximately .22 and .4. The at least one delivery waveguide may comprise a fiber having a fiber core diameter of between about 9 and 100 microns.

The at least one collection waveguide comprises a fiber having a fiber core diameter of between about 50 and 200 microns. The at least one delivery waveguide may comprise a fiber having a fiber core diameter of about 50 microns and wherein the at least one collection waveguide may comprise a fiber having a fiber core diameter of about 100 microns.

The invention also provides for a method for providing analysis and treatment of a body lumen, the method comprising: inserting into a body lumen a catheter including a flexible conduit, a lumen-expanding balloon, at least one delivery waveguide and at least one collection waveguide, a transmission output of the at least one delivery waveguide and a transmission input of the at least one collection waveguide being located within the balloon; maneuvering the conduit into a designated region of the body lumen designated for treatment or analysis; expanding the balloon in the designated region of the body lumen; executing spectroscopic analysis of the designated region of the body lumen using radiation at a wavelength in a range of about 250 to 2500 nanometers by radiating the designated region of the body lumen with the radiation that is supplied at the transmission output of the at least one delivery waveguide, the supplied radiation passing through the balloon where it is incident on the designated region of the body lumen, and wherein radiation is returned through the balloon to the transmission input of the at least one collection waveguide.

Expanding the balloon therapeutically may expand the body lumen. Expanding the balloon to therapeutically expand the body lumen may dilate the body lumen in the designated region. Executing spectroscopic analysis may be performed while the balloon is expanded. The insertion and maneuvering of the conduit and the expansion of the balloon may follow procedures in accordance with percutaneous transluminal angioplasty. The insertion and maneuvering of the conduit and the expansion of the balloon may follow procedures in accordance with percutaneous coronary transluminal angioplasty.

The balloon may be expanded such that the flow of blood between the balloon and the surrounding lumen tissue is substantially stopped. The spectroscopic analysis may include the characterization of one or more pathophysiologic or morphologic factors of surrounding tissue within an endovascular region. The pathophysiologic or morphologic factors may include characterizing the presence, volume, and positioning of plaque within the endovascular region. The pathophysiologic or morphologic factors may further include characteristics of plaque including at least one of collagen content, lipid content, calcium content, inflammation, or the relative positioning of pathophysiologic conditions within the plaque.

The method may further comprise providing a stent on the lumen-expanding balloon for delivery in the designated region at the time of expanding the balloon.

Executing spectroscopic analysis may further comprise: collecting analysis data based on the radiation that is returned through the at least one collection waveguide; and discriminating between collected analysis data associated with targeted tissue in the designated region and analysis data associated with artifacts including at least one of the balloon, a balloon expansion media, a guidewire, a stent, and an artificial material placed on a stent. The analysis data associated with artificial materials placed on stents may include data associated with polymers. The analysis data associated with artificial materials placed on stents may include data associated with drugs. The method may further comprise determining an appropriate treatment for the designated region using the spectroscopic analysis.

Determining an appropriate treatment may include selecting a type of stent most appropriate for insertion. Determining a type of stent most appropriate for insertion may include selecting a drug and dosage to be eluted from the stent. Executing spectroscopic analysis may be performed while the balloon is partially inflated. The spectroscopic analysis may be performed while the balloon is partially inflated and may be used to calculate the location of damaged tissue. The calculation of the location of damaged tissue may be used to guide the position of the conduit in the lumen prior to full inflation of the balloon.

The method may further comprise determining a level of expansion of the balloon using the spectroscopic analysis. The spectroscopic analysis may be executed on a 360 degree portion of a wall of the lumen. The executing spectroscopic analysis may include selectively switching delivery of radiation between separate ones of the at least one delivery waveguides. The selective switching may distribute radiation to radiate predefined quadrants about the circumference of the balloon. The selective switching may comprise selective operation of multiple radiation sources.

Executing spectroscopic analysis may include selectively scanning across one or more ranges of wavelengths. Executing spectroscopic analysis may include scanning using one or more ranges of wavelengths between about 750 nm and 2500 nm. The one or more ranges of wavelengths may be selected from ranges of approximately 250-930 nanometers, 1100-1385 nanometers, 1600-1850 nanometers, and 2100-2500 nanometers.

Selectively scanning across one or more ranges of wavelengths may include scanning using one or more discrete wavelengths. Expanding the balloon may comprise expanding the balloon with a biocompatible liquid that substantially minimizes the effects of scattering, distortion, and deflection of the radiation. The biocompatible liquid may be at least one selected from the group consisting of carbon dioxide, saline, deuterium oxide, and glycerin. The biocompatible liquid may comprise super-saturated saline solution.

Executing spectroscopic analysis may further comprise collecting analysis data based on the radiation that is received through the at least one collection waveguide. Collecting analysis data may occur within a time period of less than about 1 second.

The method may further comprise analyzing the collected analysis data. An amount of power emitted from the lumen-expanding balloon during the spectroscopic analysis may be less than about 20 milliwatts.

The invention also provides for a method of forming a catheter for placement within a body lumen comprising: providing a flexible conduit that is elongated along a longitudinal axis suitable for insertion into a body lumen, the flexible conduit having a proximal end and a distal end; providing at least one delivery waveguide and at least one collection waveguide along the flexible conduit, the at least one delivery waveguide and the at least one collection waveguide constructed and arranged to transmit radiation at a wavelength in a range of about 250 to 2500 nanometers; and providing a lumen-expanding inflatable balloon about a portion of the conduit so that a transmission output of the at least one delivery waveguide and a transmission input of the at least one collection waveguide are located within the balloon.

This method may further comprise forming distal portions of the at least one delivery waveguide and the at least one collection waveguide in a helical arrangement by: stripping end portions of the waveguides of outer jacketing; securing unstripped portions of the waveguides; applying a heat source to the end portions of the waveguides to be helically arranged, the heat source sufficient to make malleable the end portions; and applying forces to rotate the waveguides about a core segment and to translate longitudinally the end portions of the waveguides in the direction of their secured unstripped portions.

The waveguides may be helically arranged at predetermined angles by applying in a predetermined manner the forces to rotate and translate longitudinally the ends of the waveguides. Securing the unstripped portions of the waveguides may be performed using at least one locking member disposed about the core segment and the forces for rotating and translating are applied with a rotatably and translatably movable member disposed about the core segment. The end portions of the waveguides may be translated a distance ranging from about 2 microns to 2 millimeters, while the end portions of the waveguides are rotated about 30 to 360 degrees about the core segment.

The heat source may provide heat at about 1600 Celsius. The balloon may be laser welded to the conduit.

The method may further comprise providing a waveguide holder for contiguously retaining the at least one delivery waveguide and at least one collection waveguide to the flexible conduit. The at least one delivery waveguide and at least one collection waveguide may be assembled with said waveguide holder prior to providing said at least one delivery waveguide and the at least one collection waveguide along the flexible conduit.

The method may further comprise shaping the transmission output of said at least one delivery waveguide after the assembly with said waveguide holder. The method may further comprise shaping the transmission input of said at least one collection waveguide after the assembly with said waveguide holder. The waveguide holder for holding the at least one delivery waveguide and at least one collection waveguide may comprise a holder body having a plurality of holes. The method may further comprise aligning and fixing the plurality of holes with the longitudinal axis of the flexible conduit. The method may further comprise correspondingly aligning and fixing the plurality of holes with one or more reflective surfaces.

The one or more reflective surfaces may be disposed radially about the flexible conduit as part of a multi-faceted reflecting element. The or more reflective surfaces may comprises a cone- shaped reflecting element aligned with the longitudinal axis of the flexible conduit.

The waveguide holder for holding the at least one delivery waveguide and at least one collection waveguide may comprise a holder body having a plurality of grooves disposed radially about the flexible conduit.

The invention also provides for a catheter for placement within a body lumen, the catheter comprising: a flexible conduit that is elongated along a longitudinal axis, the flexible conduit having a proximal end and a distal end; at least one delivery waveguide and at least one collection waveguide positioned along the flexible conduit, the at least one delivery waveguide and the at least one collection waveguide constructed and arranged to transmit radiation at a wavelength in a range of about 250 to 2500 nanometers; and a lumen-expanding inflatable balloon disposed about a portion of the conduit, a transmission output of the at least one delivery waveguide and a transmission input of the at least one collection waveguide being positioned along an outer surface of the balloon.

The catheter may further comprise a ring that couples body portions of the at least one delivery waveguide and the at least one collection waveguide to the flexible conduit. The transmission output of the at least one delivery waveguide and a transmission input of the at least one collection waveguide may be mounted on the outer surface of the balloon.

The invention also relates to a catheter for placement within a body lumen, the catheter comprising: a flexible conduit that is elongated along a longitudinal axis, the flexible conduit having a proximal end and a distal end; at least one delivery waveguide and at least one collection waveguide positioned along the flexible conduit, the at least one delivery waveguide and the at least one collection waveguide constructed and arranged to transmit radiation at a wavelength in a range of about 250 to 2500 nanometers; and a lumen-expanding inflatable balloon disposed about a portion of the conduit, a transmission output of the at least one delivery waveguide and a transmission input of the at least one collection waveguide being mounted to an inner surface of the balloon.

The catheter may further comprise a ring that couples body portions of the at least one delivery waveguide and the at least one collection waveguide to the flexible conduit

## Claims

1. A system for probing and treating a body lumen comprising:
a flexible conduit that is elongated along a longitudinal axis suitable for insertion into a body lumen, the conduit having a proximal end and a distal end;
at least one delivery waveguide and at least one collection waveguide integrated with the flexible conduit;
at least one radiation source connected to a transmission input of the at least one delivery waveguide, the radiation source constructed and arranged to provide radiation at a wavelength in a range of about 750 to 2500 nanometers;
at least one optical detector connected to a transmission output of the at least one collection waveguide wherein the transmission output of the at least one collection waveguide is connected to a spectrometer, the spectrometer constructed and arranged to scan radiation and perform spectroscopy at the wavelength in the range of about 750 nm to 2500 nm; and
a lumen-expanding inflatable balloon disposed about a portion of the conduit, a transmission output of the at least one delivery waveguide and a transmission input of the at least one collection waveguide adjacent to the outside surface of the balloon, and the collection fibers arranged to receive reflected optical radiation from the lumen wall.

2. The system of claim 1 wherein the spectrometer is configured to perform spectroscopy selected from the group of spectroscopy methods consisting of fluorescence, light scatter, optical coherence reflectometry, optical coherence tomography, speckle correlometry, Raman, and diffuse reflectance spectroscopy.

3. The system of claim 2 wherein the spectrometer is configured to perform spectroscopy consisting of diffuse reflectance spectroscopy.

4. The system of claim 1 wherein a scan includes one or more wavelengths of between about 1100 and 1385 nm.

5. The system of claim 1 comprising a controller that is programmed to operate a human-interactive interface that provides an operator with feedback about data and analysis of the spectroscopy, the interface providing information for real-time diagnosis.

6. The system of claim 1 comprising a controller that is programmed to identify one or more characteristics of targeted tissue including tissue morphological structures.

7. The system of claim 1 wherein comprising a controller that is further programmed to discriminate between tissue characteristics and stents.

8. The system of claim 1 further comprising a switch coupled between the at least one radiation source and the at least one delivery waveguide that selects between multiple radiation sources for application of radiation to the at least one delivery waveguide.

9. The system of claim 1 wherein the lumen-expanding balloon comprises an angioplasty balloon.

10. The system of claim 1 further comprising an optical element disposed about the flexible conduit, the optical element including an array of multiple facets that lie at an acute angle relative to the longitudinal axis of the flexible conduit for changing a direction of radiation transmitted along a longitudinal axis of the at least one delivery or collection waveguide so that the radiation is emitted in a direction or received from a direction that is transverse to the longitudinal axis of the at least one delivery or collection waveguide, respectively.

11. The system of claim 1 wherein the transmission output of the at least one delivery waveguide and the transmission input of the at least one collection waveguide are spaced apart at a predetermined distance in a longitudinal direction along the longitudinal axis of the conduit.

12. The system of claim 1 wherein the at least one delivery waveguide comprises two, three or four delivery waveguides and wherein the at least one collection waveguide comprises two, three or four collection waveguides.

13. The system of claim 1 wherein the plurality of transmission outputs of the plurality of delivery waveguides are arranged to execute spectroscopic analysis on a 360 degree portion of a wall of the lumen.

14. The system of claim 1 wherein the at least one delivery waveguide comprises a fiber having a fiber core diameter of between about 9 and 100 microns and the at least one collection waveguide comprises a fiber having a fiber core diameter of between about 50 and 200 microns.

15. The system of claim 1 wherein a maximum outer diameter of the flexible conduit, the at least one delivery waveguide, the at least one collection waveguide and the balloon is less than about 1.5 millimeters when the balloon is uninflated.
